# EUROPEAN PATENT APPLICATION

(11) **EP 4 484 408 A1**
(43) Date of publication of application: **01.01.2025**
(21) Application number: 23773710.1
(22) Date of filing: 17.03.2023
(51) Int. Cl.: C07C 219/16, C07C 229/12, C07C 271/20, C07C 333/04, C07C 329/06, C07C 271/16, C07C 271/22, C07C 269/00, C07C 269/04, C07C 227/00, A61K 47/18, A61K 47/20, A61K 9/127

(54) **CATIONIC LIPID COMPOUND, PREPARATION METHOD THEREFOR AND USE THEREOF, AND MRNA DELIVERY SYSTEM**

(30) Priority: 25.03.2022 CN 202210301173; 06.03.2023 CN 202310203981
(71) Applicant: Shenzhen Neocurna Biotechnology Corporation, Shenzhen, Guangdong 518110 (CN); Neocura Bio-Medical Technology Co., Ltd., Shenzhen, Guangdong 518000 (CN); Beijing Neocurna Biotechnology Corporation, Beijing 102200 (CN)
(72) Inventor: ZHAO, Zhao, Shenzhen, Guangdong 518110 (CN); SU, Tao, Shenzhen, Guangdong 518110 (CN); LI, Hongyan, Shenzhen, Guangdong 518110 (CN); LI, Sanpeng, Shenzhen, Guangdong 518110 (CN); GUO, Fengjuan, Shenzhen, Guangdong 518110 (CN); HE, Huamei, Shenzhen, Guangdong 518110 (CN); WAN, Ji, Shenzhen, Guangdong 518110 (CN); PAN, Youdong, Shenzhen, Guangdong 518110 (CN); WANG, Yi, Shenzhen, Guangdong 518110 (CN)
(74) Representative: Huang, Liwei
(86) International application number: PCT/CN2023/082055
(87) International publication number: WO 2023/179462

(57) **Abstract**

Disclosed in the present invention are a cationic lipid compound, a preparation method and a use thereof, and a delivery system for mRNA, which solve the following problem: there is an imminent need for developing a cationic lipid having high delivery efficiency and excellent immune activation characteristics. The cationic lipid compound has a structure represented by formula (I): 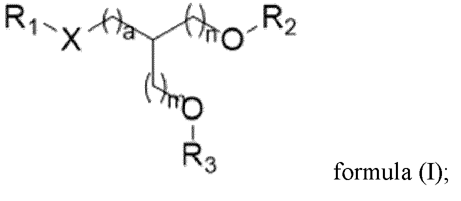 wherein, in the formula (I): X is O or N; n is 2-4; m is 2-4; a is 0 or 1; R₁ is a chain structure comprising a tertiary amine; R₂ is a linear fatty acyl group or a branched chain fatty acyl group; R₃ is a branched chain fatty acyl group. The present invention is principally used to develop a cationic lipid compound, which is used for preparing a delivery system for mRNA, and has the characteristics of high delivery efficiency, organ-targeted delivery and excellent immune activation characteristics.

## Description

### CROSS-REFERENCE TO RELATED APPLICATION

This disclosure claims priority to Chinese Patent Application No. 2022103011731 filed to China National Intellectual Property Administration (CNIPA) on March 25, 2022 and entitled "CATIONIC LIPID COMPOUND, PREPARATION METHOD AND USE THEREOF, AND DELIVERY SYSTEM FOR MRNA", and claims priority to Chinese Patent Application No. 202310203981 filed to China National Intellectual Property Administration (CNIPA) on March 6, 2023 and entitled "CATIONIC LIPID COMPOUND, PREPARATION METHOD AND USE THEREOF, AND DELIVERY SYSTEM FOR MRNA", the contents of which are incorporated herein by reference in their entireties.

### TECHNICAL FIELD

The present invention belongs to the field of pharmaceutical biotechnology, and in particular, relates to a cationic lipid compound, a preparation method and a use thereof, and a delivery system for mRNA.

### BACKGROUND

Messenger ribonucleic acid (mRNA) is a type of single-stranded ribonucleic acid, which is obtained by polymerizing four kinds of ribonucleoside triphosphates (ATP, UTP, GTP, and CTP) as substrates via phosphodiester bonds under the catalytic action of RNA polymerase by using one strand of a double strand of a deoxyribonucleic acid (DNA) as a template. mRNA can carry and transmit the genetic information stored by DNA in a cell nucleus, which plays a key role in the conversion of the genetic information into functional proteins. In cytoplasm, immature mRNA is processed and modified into mature mRNA through steps such as capping, tailing, intron splicing, and the like. The mature mRNA can accurately guide the synthesis process of proteins in the cytoplasm. Comparatively speaking, since mRNA is relatively much smaller in molecular weight than the DNA, mRNA is easy to transfect and has no carcinogenic risk of insertion mutation caused by integration into host DNA. Therefore, mRNA is used as a preventive and therapeutic drug and has great advantages and potentials in the prevention and treatment of various diseases.

For mRNA-based drugs, a target functional gene or a functional subunit of a target gene is introduced into a patient's body in the form of the messenger ribonucleic acid by molecular biological methods, and proteins with specific functions are expressed through targeted intracellular delivery, escape from late endosomes, intracellular translation and modification by post-translational processing, which is a preventive and therapeutic strategy used to prevent (functional proteins or subunits activate a host's immune system to produce corresponding humoral or cellular immune responses) or treat diseases (expressed proteins or subunits have the function of treating diseases or regulating the expressions of other genes).

mRNA-based drugs have the advantages that they can directly activate the body to produce functional antibodies or cellular immune responses against specific pathogens, repair pathogenic genes or correct abnormal gene expression at the molecular level, thus achieving the effects of preventing and treating various diseases. mRNA-based drugs can achieve the effects that the traditional drugs cannot replace, for example, monoclonal antibody drugs can merely act on the cell surface, while mRNA-based drugs can act not only on extracellular proteins, but also act on intracellular proteins, even act in the cell nucleus, and have accurate targeting property. Among over 7000 diseases faced by human beings, about 1/3 of them are caused by the abnormal expression (gene deletion, reduction or overexpression) of functional genes, such as hemophilia, Duchenne muscular dystrophy (DMD), cystic fibrosis, and severe combined immunodeficiency (SCID), which are almost clinically incurable. However, mRNA-based drugs are especially advantageous to such single-gene diseases. In the era of the popularization of personalized medicine and precision medicine, theoretically, all diseases caused by gene differences or gene abnormal expression of patients can be accurately and effectively treated with mRNA-based drugs.

mRNA-based drugs have great advantages and potential in regulating gene expression and in the prevention and treatment of malignant diseases. However, there are numerous difficulties in the research and development, preparation and subsequent system administration of such drugs. Firstly, mRNA exists in a single-strand form, resulting in mRNA being extremely unstable in vitro and under physiological conditions, and is not only easily degraded by RNA nuclease (RNase) in air or blood, but also easily cleared by mononuclear macrophages in in tissues and organs such as the liver and/or spleen. Secondly, since mRNA is negatively charged, it is difficult for mRNA to enter into the cell through the cell membrane. Thirdly, it is difficult for mRNA to escape from endosomes and enter into the cytoplasm to play its role. In addition, uracil ribonucleotide (U) in mRNA is easy to cause immunogenicity, and in some cases, the immunogenicity may increase the potential toxic side effects of mRNA-based drugs. Finally, the common occurrence of off-target effects is also an important challenge in the preparation and administration of mRNA-based drugs. Therefore, the development of intracellular delivery systems for mRNA-based drugs is the key to its large-scale clinical application. Herein, since cationic lipid is an important component of delivery system for mRNA (LNP), there is an imminent need for developing a cationic lipid having high delivery efficiency and excellent immune activation characteristics.

### SUMMARY

In view of the above, the present invention provides a cationic lipid compound, a preparation method and a use thereof, and a delivery system for mRNA, and the main objective thereof is to develop a cationic lipid compound for preparing a delivery system for mRNA, which has the characteristics of high delivery efficiency, organ-targeted delivery, and excellent immune activation characteristics.

In order to achieve the above purpose, the present invention mainly provides the following technical solutions:

In one aspect, an embodiment of the present invention provides a cationic lipid compound, wherein the cationic lipid compound has a structure represented by the following formula (I): 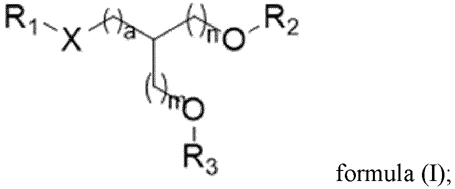 wherein, in the formula (I): X is O or N; n is 2-4; m is 2-4; a is 0 or 1; R₁ is a chain structure comprising a tertiary amine; R₂ is a linear fatty acyl group or a branched chain fatty acyl group; R₃ is a branched chain fatty acyl group.

Preferably, the chain structure comprising a tertiary amine is a chain structure of a tertiary amine, preferably is selected from any one of the groups consisting of:

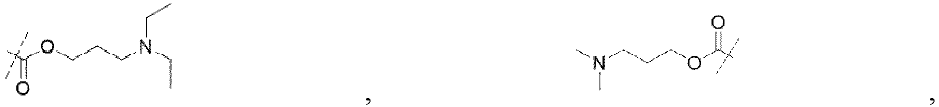

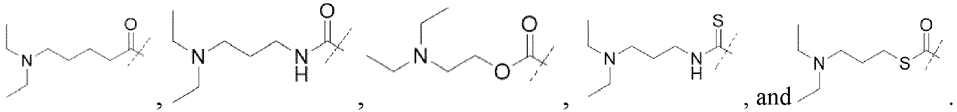

Preferably, the linear fatty acyl group is a C8-C16 linear fatty acyl group, preferably a C10-C14 linear fatty acyl group, more preferably selected from any one of the groups consisting of:

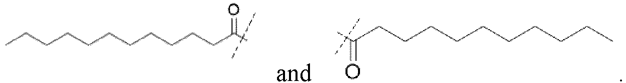

Preferably, in the branched chain fatty acyl group: the number of the long-chain C atoms is less than or equal to 16, and the number of the short-chain C atoms is less than or equal to the number of the long-chain C atoms; preferably, the branched chain fatty acyl group; further preferably, the branched chain fatty acyl group is selected from any one of the groups consisting of:

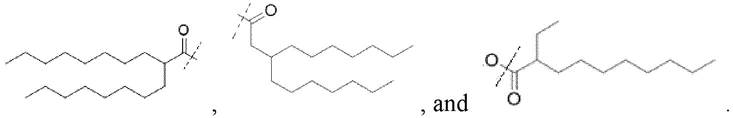

Preferably, the cationic lipid compound is any one of the following compounds:

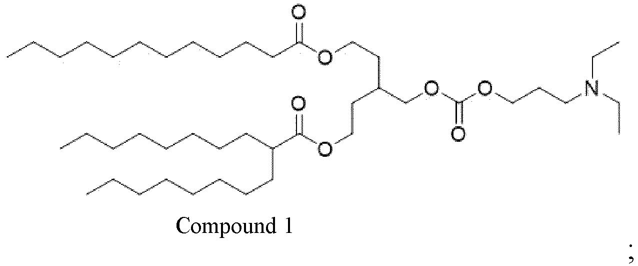

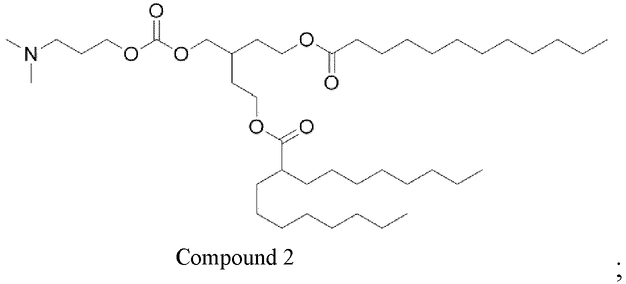

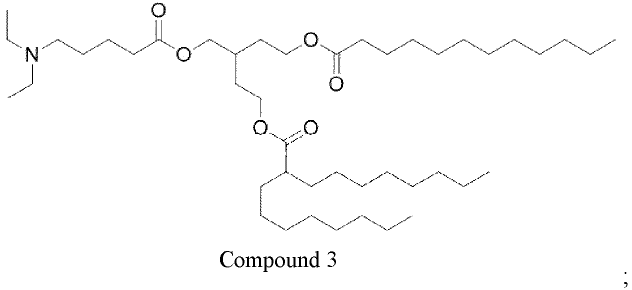

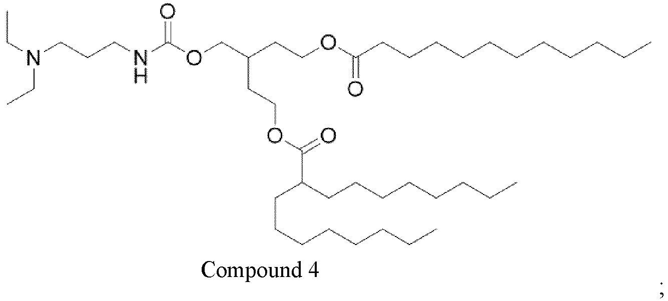

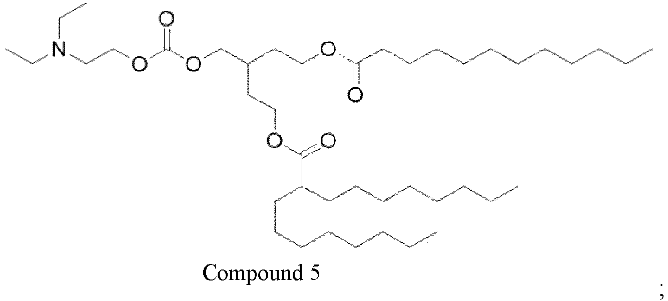

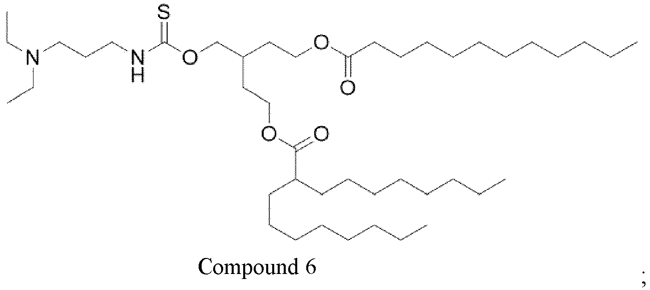

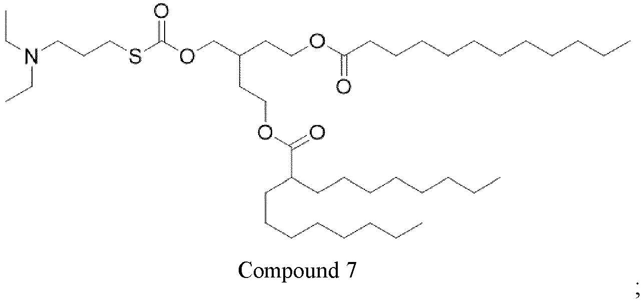

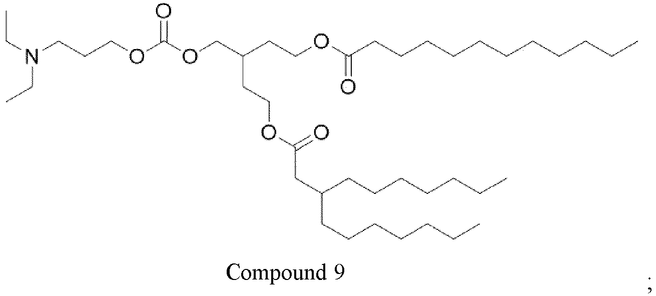

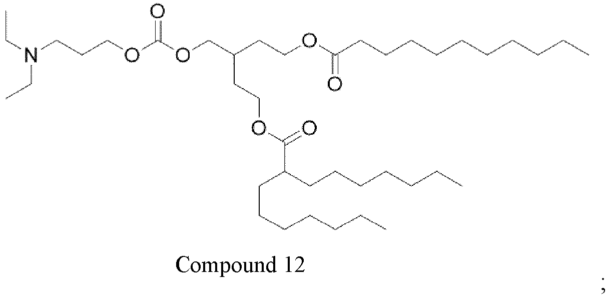

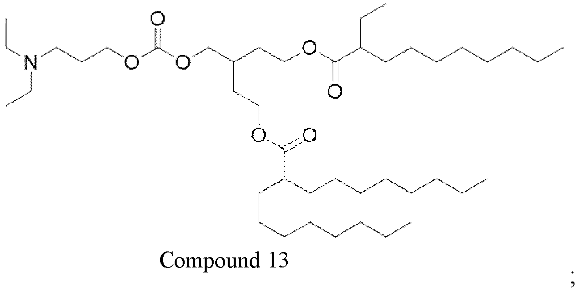

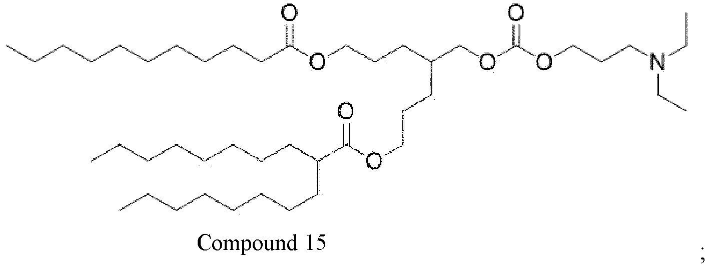

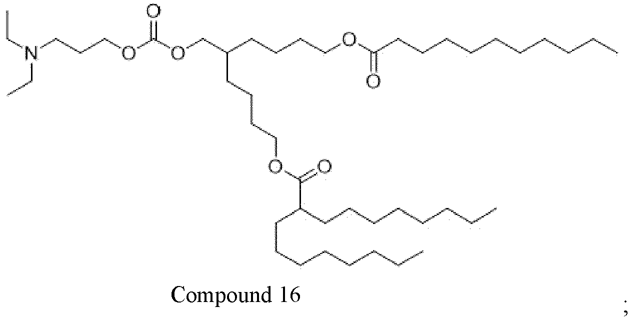

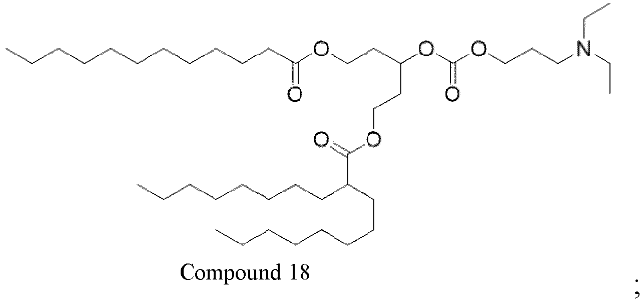

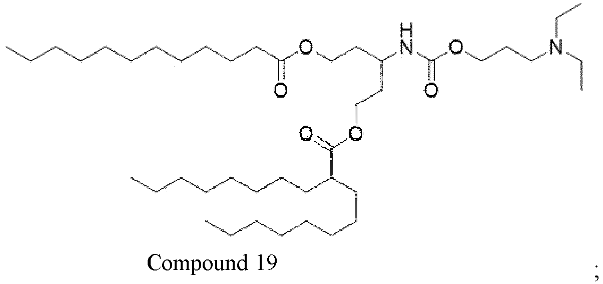

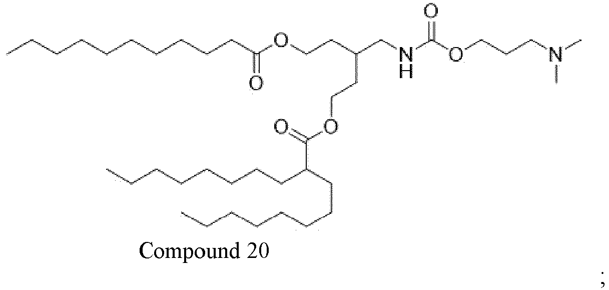

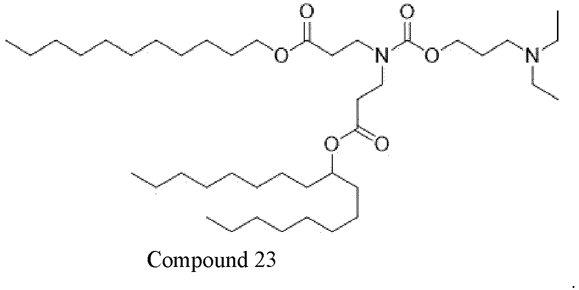

Preferably, the chemical reaction procedure for preparing the Compounds 1-5 and 7 is as follows:

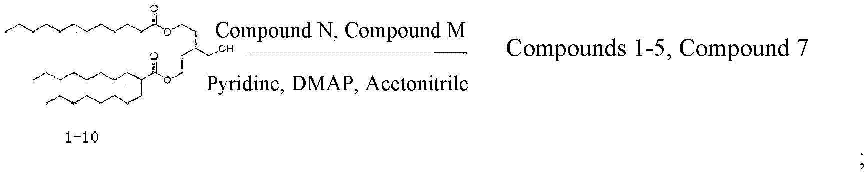

when the prepared compound is the Compound 1, the Compound N and the Compound M are selected as: 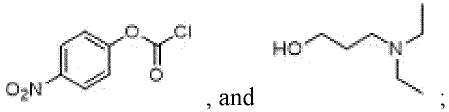 when the prepared compound is Compound 2, the Compound N and the Compound M are selected as: 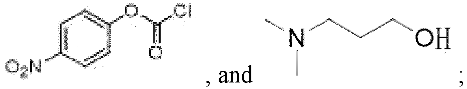 when the prepared compound is Compound 3, the Compound N and the Compound M are selected as: 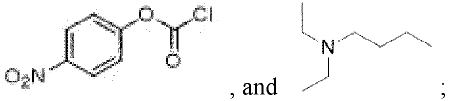 when the prepared compound is Compound 4, the Compound N and the Compound M are selected as: 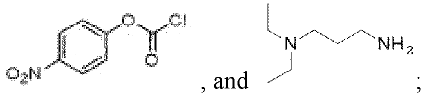 when the prepared compound is Compound 5, the Compound N and the Compound M are selected as: 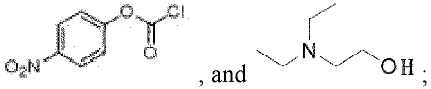 when the prepared compound is Compound 7, the Compound N and the Compound M are selected as:

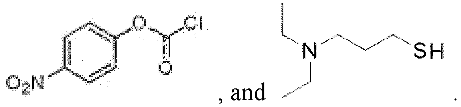

Preferably, the preparation steps of the Compound 1 comprise: first, raw material 1-10, pyridine, 4-dimethylaminopyridine and acetonitrile are added into a reaction vessel under room temperature conditions; and then further p-nitrophenyl chloroformate is added thereto for reaction, wherein after the reaction is carried out for a set time, a Thin Layer Chromatography (TLC) spot plate shows that a new main spot is formed; and then further 3-diethylamino-1-propanol is added into the reaction system for reaction; after the reaction is completed, extraction and liquid separation are carried out; the organic phase is washed, dried and purified to obtain a colorless oily product Compound 1.

Preferably, the chemical reaction procedure for preparing the raw material 1-10 is as follows:

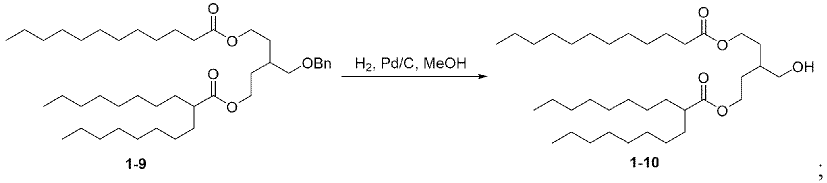

Preferably, the preparation steps of the raw material 1-10 comprise: raw material 1-9, methanol and palladium-carbon containing water are added into a reaction vessel under room temperature conditions; after the reaction vessel is sequentially replaced with inert gas and hydrogen gas for multiple times, a reaction is carried out; after the reaction is completed, a suction filtration treatment is carried out to remove palladium-carbon; the filtrate is spin-dried to obtain a colorless oily product as raw material 1-10.

Preferably, the chemical reaction procedure for preparing the Compound 9, the Compound 12, the Compound 13, the Compound 15, the Compound 16 and the Compound 18 is as follows: 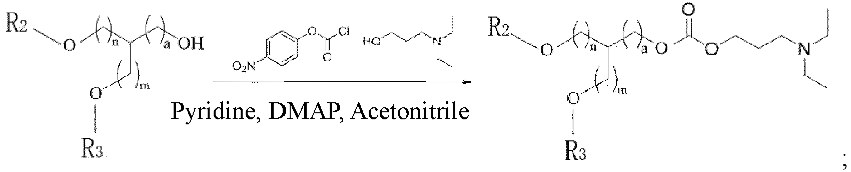 wherein the preparation steps comprise: raw materials 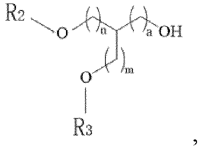 pyridine, 4-dimethylaminopyridine, and acetonitrile are added into a vessel under room temperature conditions; after dissolution, p-nitrophenyl chloroformate is added thereto for reaction; the reaction is monitored by TLC, until a new fluorescent spot is generated; and then 3-dimethylamino-1-propanol is added into the reaction system for reaction; after the reaction is completed, the extraction and liquid separation are carried out, and the organic phase is washed, dried and purified to obtain the required compound.

Preferably, the chemical reaction procedure for preparing the compounds 19 and20 is as follows:

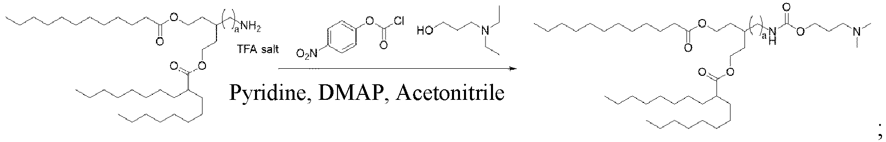

Preferably, the preparation steps of the compounds 19 and20 comprise: raw materials 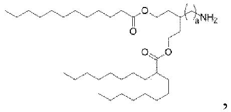 pyridine, 4-dimethylaminopyridine, and acetonitrile are added into a reaction vessel; after sufficiently dissolving, p-nitrophenyl chloroformate is added thereto for reaction; the reaction is monitored by TLC, until a new fluorescent spot is generated; and then 3-dimethylamino-1-propanol is added into the reaction system for reaction overnight; after the reaction is completed, the extraction and liquid separation are carried out, and the organic phase is washed, dried and purified to obtain the compounds 19 and20.

Preferably, the chemical formula for preparing the Compound 6 is as follows: 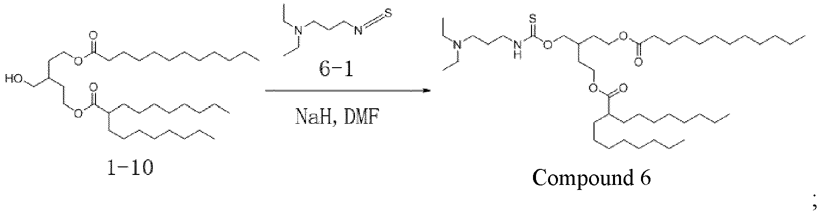 wherein the preparation steps of the Compound 6 are as follows: raw material 1-10 and DMF are added into a reaction vessel, mixed and dissolved, and then cooled to 0-5 °C under an ice-water bath condition, and then sodium hydride is added into the reaction vessel in batches for reaction, and after the reaction is carried out for a set time, raw material 6-1 are dropwise added into the reaction system for reaction; after the reaction is completed, quenching, the extraction and liquid separation, drying, and purification are sequentially carried out to obtain the colorless oily Compound 6.

Preferably, the chemical formula for preparing the Compound 23 is as follows:

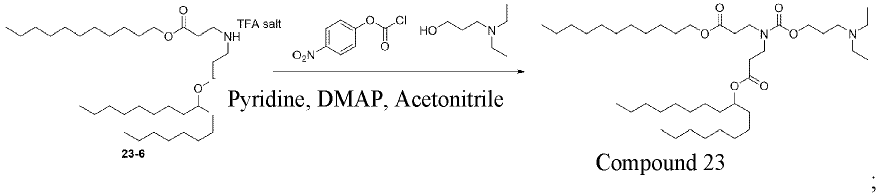

Preferably, the preparation steps of the Compound 23 comprise: raw material 23-6, pyridine, 4-dimethylaminopyridine and acetonitrile are added into a reaction vessel under room temperature conditions; after full dissolution, p-nitrophenyl chloroformate is added thereto for reaction, wherein the reaction is monitored by TLC, until a new fluorescent spot is generated; and then 3-dimethylamino-1-propanol is added into the reaction system for reaction overnight; after the reaction is completed, the extraction and liquid separation are carried out, and the organic phase is washed, dried and purified to obtain the Compound 23.

In a further aspect, the present invention provides a use of the cationic lipid compound according to any one of the above in the preparation of a delivery system for mRNA.

In a yet another aspect, an embodiment of the present invention also provides a delivery system for mRNA, wherein the delivery system for mRNA comprises the cationic lipid compound according to any one of the above.

Compared with the prior art, the cationic lipid compound, the preparation method and use thereof, and the delivery system for mRNA according to the present application have at least the following beneficial effects:

On the one hand, the embodiment of the present invention develops a novel cationic lipid compound having a structure represented by formula (I), and the cationic lipid compound is used for preparing a delivery system for mRNA and has the characteristics of high delivery efficiency, organ targeted delivery, excellent immune activation characteristic and good safety.

On the other hand, the embodiment of the present invention provides a delivery system for mRNA (LNP), which has the characteristics of high delivery efficiency, organ targeted delivery, excellent immune activation characteristic and good safety because the delivery system for mRNA comprises the above-mentioned cationic lipid compound.

The foregoing description is only an overview of the technical solution of the present invention, and is intended to provide a better understanding of the technical solution of the present invention, as it is embodied in the following description, with reference to the preferred embodiments of the present invention and the accompanying drawings.

### BRIEF DESCRIPTION OF DRAWINGS

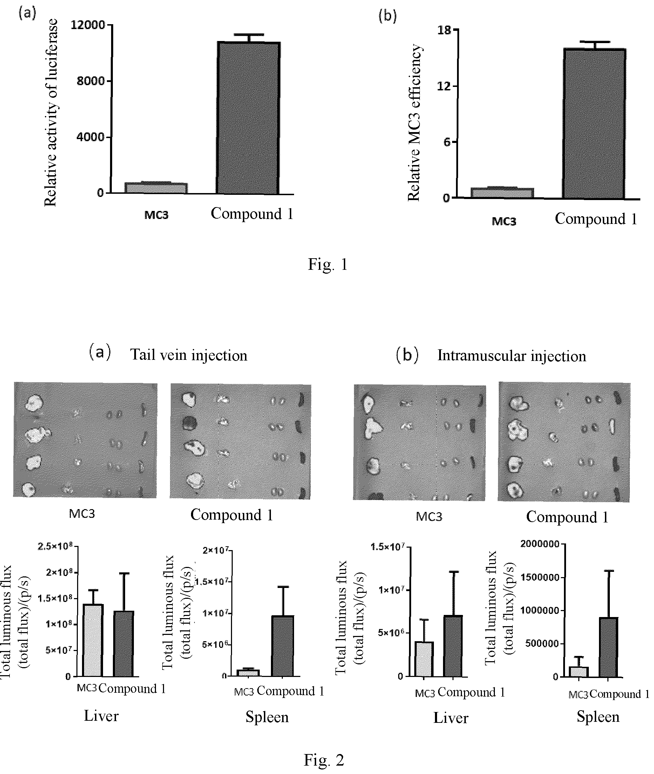

FIG. 1 shows cell transfection efficiency for lipid nanoparticle formulation prepared from Compound 1 of the present invention and a control; wherein, FIG. (a) shows the relative luciferase activities after transfection of cells with the lipid nanoparticle formulation prepared from Compound 1 and the control nanoparticle; FIG. (b) shows the normalized results of the relative luciferase activities after transfection of cells with two lipid nanoparticles.
FIG. 2 shows in vivo transfection efficiency of lipid nanoparticle formulation prepared from Compound 1 of the present invention and a control; wherein, FIG. (a) is a graph showing luciferase distribution of main organ and shows the quantitative results of liver and spleen distribution measured after 6 hours of tail vein injection of lipid nanoparticle in mouse; FIG. (b) is a graph showing luciferase distribution of main organ and shows the quantitative results of liver and spleen distribution in mice after 6 hours of intramuscular injection of lipid nanoparticle.

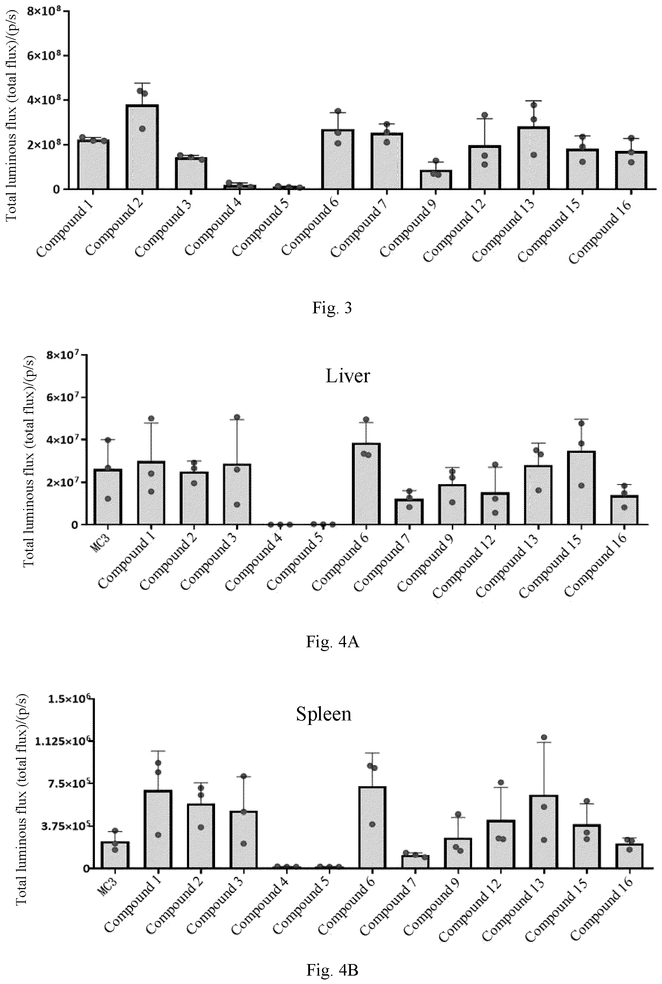

FIG. 3 shows in vivo transfection efficiency of lipid nanoparticle formulation prepared from compounds of the present invention and a control.

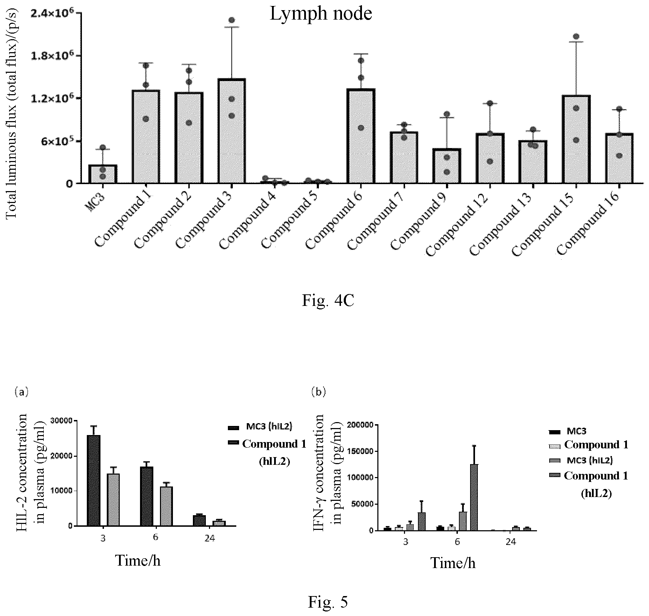

FIGS. 4A, 4B, and 4C show transfection effects at different sites of lipid nanoparticle formulations prepared from compounds of the present invention and a control; wherein, FIG. 4A shows the quantitative results of liver distribution in mice after 6 hours intramuscular injection; FIG. 4B shows the quantitative results of spleen distribution in mice after 6 hours intramuscular injection; FIG. 4C shows the quantitative results of lymph node distribution in mice after 6 hours intramuscular injection.
FIG. 5 shows the immune activation effect of transfection of hIL-2 mRNA in vivo of lipid nanoparticle formulation prepared from Compound 1 of the present invention and a control; wherein, FIG. (a) shows the hIL-2 concentration in plasma; and FIG. (b) shows the IFN-γ concentration in plasma.

### DETAILED DESCRIPTION

In order to further explain the technical means and effects adopted by the application to achieve its intended purpose, the specific implementation mode, structure, characteristics and effects of the remote video monitoring system for a communication cabinet provided by the application will be described in detail below with reference to the drawings and preferred embodiments. In the following description, different "one embodiment" or "an embodiment" do not necessarily refer to the same embodiment. In addition, a particular characteristic, structure or feature in one or more embodiments may be combined in any suitable form.

The preparation method of the cationic lipid compound of the present invention is described in detail herein, and specifically as follows:
1.

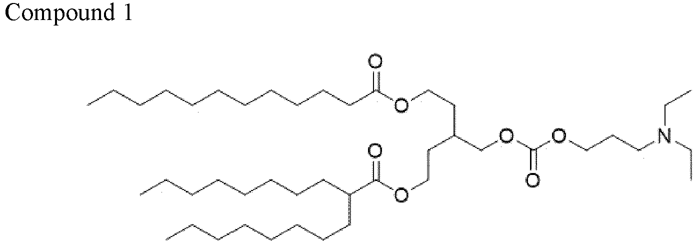

The preparation method of the Compound 1 is as follows:

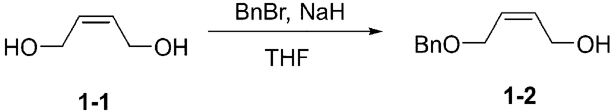

In a 500mL three-necked flask in which a thermometer was inserted, cis-2-Butene-1,4-diol 1-1 (raw material 1-1, 33.0g, 0.375mol) and anhydrous tetrahydrofuran (300 mL for dissolving raw material 1-1) were added under ice-water bath conditions, respectively; after cooling to 0-5 °C, the weighed sodium hydride (60% content, 0.45mol,18.0 g) was added slowly in portions to the reaction system, after a reaction was performed for 30 minutes, benzyl bromide (63.75 g, 0.375 mol) was added to the reaction system through a constant pressure dropping funnel, after the dropwise adding was finished, a reaction was carried out for 2 hours, in TLC (petroleum ether: ethyl acetate=5:1) potassium permanganate developed to show that the raw materials were completely reacted, and after a new main spot was generated, the reaction was stopped. To the reaction solution, a saturated ammonium chloride solution was slowly added for quenching, and then ethyl acetate was added for the extraction and liquid separation treatment, and the organic phase was dried and spin-dried to obtain a light-yellow oily liquid. Then, a silica gel was added for mixing. A colorless oily product 1-2 (34 g, 50.9%) was obtained by column chromatography (petroleum ether: ethyl acetate=20:1 to 5:1).

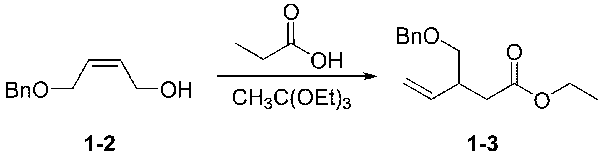

Raw material 1-2 (34 g, 0.19 mol), n-propionic acid (4.94 g, 0.07 mol), and triethyl orthoformate (184.7 g, 1.14 mol) were added separately in a 250mL sealed tube at room temperature, mixed well and sealed, and then the temperature was raised to 150 °C. After the reaction was carried out for 3 hours, a reaction mixture was cooled. According to monitoring by a TLC spot plate, it showed that the raw material was completely reacted. Then, ethyl acetate (500 mL) and water (500 mL) were added for the extraction and liquid separation. The organic phase was washed 2-3 times with sodium bicarbonate solution (300 mL) until no bubbles emerged. The organic phase was dried and spin-dried, and then silica gel was added for mixing. A light-yellow oily product 1-3 (33 g, 69.7%) was obtained by column chromatography (petroleum ether: ethyl acetate = 10:1 to 4:1).

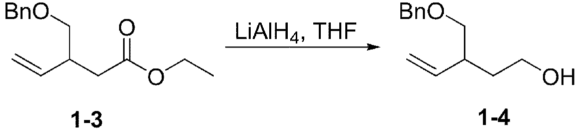

Raw material 1-3 (33.0 g, 0.133mol, 1.0 eq.) and anhydrous tetrahydrofuran (300 mL) were added in a 500mL three-necked flask under ice-water bath conditions, and then lithium aluminum tetrahydrogen solid (6.87 g,0.146 mol) was carefully added in portions. After the addition was completed, the temperature of the reaction solution was slowly raised to room temperature, and after 2 hours of reaction, according to monitoring by a TLC, it showed that the raw material was completely reacted. Then, ethyl acetate (50 mL) was added to the reaction solution to dilute, and further water (6.8 mL), a 15% NaOH aqueous solution (6.8 mL) and water (20 mL) were slowly added to the reaction solution to quench, and then suction-filtered. The filter cake was washed 3 times with ethyl acetate (50 mL); the organic phases were combined and spin-dried, followed by adding a silica gel for mixing. A light-yellow oily product 1-4 (17 g, 62%) was obtained by column chromatography (dichloromethane: methanol=50:1 to 20:1).

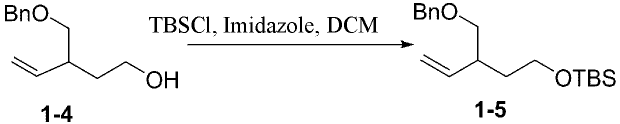

Raw material 1-4 (17.0 g, 82.5 mmol), imidazole (8.42 g, 123.8 mmol) and anhydrous dichloromethane were added in a 250 mL single-necked flask under room temperature conditions, and then t-butyldimethylchlorosilane (14.85 g, 99 mmol) was slowly added. After the dropwise addition was completed, the reaction was carried out overnight. According to monitoring by TLC, it showed that most of the raw material was completely reacted. Then, dichloromethane (200 mL) and water (200 mL) were added to the reaction for the extraction and liquid separation; The organic phase was washed 2-3 times with saturated ammonium chloride, dried and spin-dried. Then, silica gel was added for mixing. A light-yellow oily product 1-5 (14 g, 53.0%) was obtained by column chromatography (petroleum ether: ethyl acetate = 20:1 to 10:1).

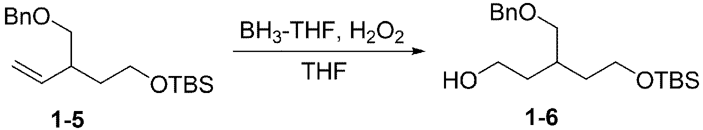

Raw material 1-5 (13.0 g, 40.6 mmol) and anhydrous tetrahydrofuran (100 mL) were added in a 250 mL three-necked flask at room temperature. After vacuumizing and replacing nitrogen for 3 times, borane dimethyl sulfide solution (10 mol/L, 6.1 mL, 60.9 mmol) was added. After reacting at room temperature for 3 hours, TLC monitoring showed that the raw material spot has disappeared. Then, 3 mol/mL NaOH solution (13.5 mL, 40.6 mmol) and hydrogen peroxide (37% content, 13.5 mL) were slowly added for reaction overnight. TLC monitoring showed that most of the raw material was completely reacted. Then, saturated sodium bisulfite solution was added for quenching, and ethyl acetate (100 mL) and water (100 mL) were used for the extraction and liquid separation. The organic phase was washed with saturated ammonium chloride 2-3 times, dried, and spin-dried. Then, silica gel was added for mixing. A colorless oily product 1-6 (9 g, 60.8%) was obtained by column chromatography (petroleum ether: ethyl acetate = 5:1 to 2:1).

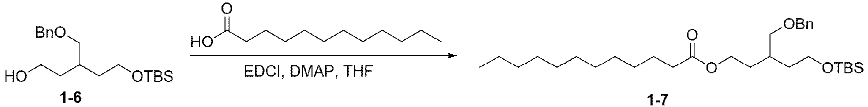

Raw material 1-6 (9.0 g, 26.6 mmol), dodecanoic acid (6.4 g, 32.0 mmol), 1-(3-dimethylaminopropyl)-3-ethylcarbodiimide hydrochloride (6.1 g, 32.0 mmol), N,N-dimethylpyridine (3.9 g, 32.0 mmol) and dichloromethane (150 mL) were added in a 250 mL single-necked flask at room temperature for reaction overnight. TLC monitoring showed that most of the raw material was completely reacted; Then, ethyl acetate (200 mL) and water (200 mL) were added to the reaction solution for the extraction and liquid separation. The organic phase was washed with saturated ammonium chloride 2-3 times, dried and spin-dried. Then, silica gel was added for mixing. A colorless oily product 1-7 (13 g, 94%) was obtained by column chromatography (petroleum ether: ethyl acetate = 50:1 to 20:1).

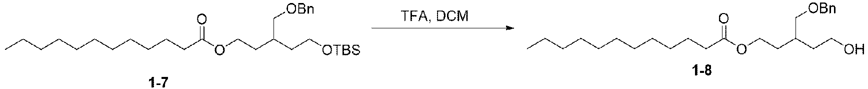

Raw material 1-7 (13.0 g, 25.0 mmol) and dichloromethane solution (150 mL) were added in a 500 mL single-necked flask at room temperature, and then trifluoroacetic acid (50 mL) was added to react for 1 hour. TLC monitoring showed that most of the raw material was completely reacted; The reaction solution was spin-dried, and then ethyl acetate (100 mL) and water (100 mL) were added thereto for the extraction and liquid separation. The organic phase was washed with saturated sodium bicarbonate 2-3 times, dried and spin-dried. Then, silica gel was added for mixing. A colorless oily product 1-8 (9.0 g, 88.7%) was obtained by column chromatography (petroleum ether: ethyl acetate = 10:1 to 5:1).

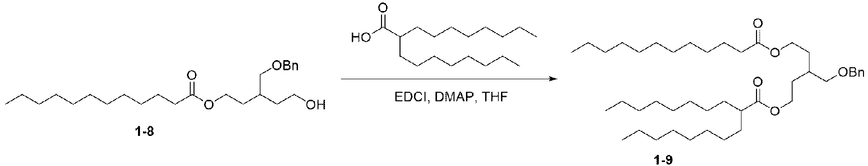

Raw material 1-8 (9.0 g, 22.1 mmol), 2-octyldecanoic acid (7.55 g, 26.6 mmol), 1-(3-dimethylaminopropyl)-3-ethylcarbodiimide hydrochloride (5.1 g, 26.6 mmol), 4-dimethylaminopyridine (3.27 g, 26.6 mmol) and dichloromethane (150 mL) were added in a 500 mL single-necked flask at room temperature for reaction overnight. TLC monitoring showed that most of the raw material was completely reacted. Then, ethyl acetate (200 mL) and water (200 mL) were added to the reaction solution for the extraction and liquid separation. The organic phase was washed with saturated ammonium chloride 2-3 times, dried, and spin-dried. Then, silica gel was added for mixing. A light-yellow oily product 1-9 (12 g, 80.5%) was obtained by column chromatography (petroleum ether: ethyl acetate = 100:1 to 20:1).

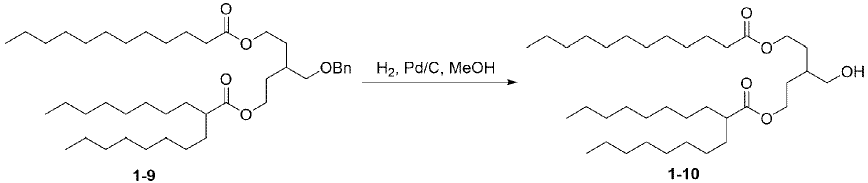

Raw material 1-9 (12.0 g, 17.8 mmol), methanol (100 mL), and palladium carbon containing water (5% palladium content, 1.2 g, 10 wt%) were added in a 250 mL single-necked flask at room temperature. The atmosphere in the flask was replaced with nitrogen three times. Then, a hydrogen-filled balloon was attached. After being replaced with hydrogen for 3 times, the reaction was allowed to proceed overnight. TLC monitoring showed that the raw material was completely reacted. Then, diatomaceous earth was used for suction filtration to remove palladium carbon. The filtrate was spin-dried to obtain a colorless oily product 1-10 (10 g, 96.1%). The crude product was used directly in subsequent reactions without further purification.

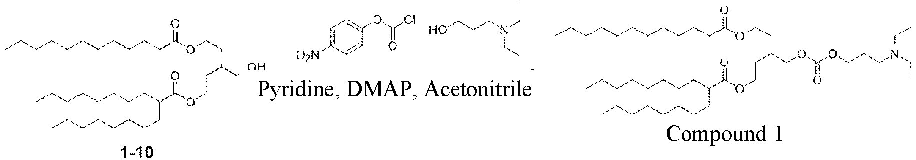

Raw material 1-10 (1.0 g, 1.71 mmol), pyridine (0.27 g, 3.42 mmol), 4-dimethylaminopyridine (41 mg, 0.34 mmol) and acetonitrile (50 mL) were added in a 100 mL single-necked flask at room temperature. Then, p-nitrophenyl chloroformate (515 mg, 2.56 mmol) was added first. After reacting for 2 hours, TLC spot plate showed that a new main spot was formed. Then, 3-diethylamino-1-propanol (448 mg, 3.42 mmol) was added to the reaction system for reaction overnight. LCMS monitoring showed that the product was generated. Then, ethyl acetate (100 mL) and water (100 mL) were added to the reaction solution for the extraction and liquid separation. The organic phase was washed with saturated ammonium chloride for 2-3 times, dried and spin-dried to obtain 2.27 g of a crude product. Then, a batch of raw materials were added repeatedly (in total: 2 g) and mixed. Then, silica gel was added for mixing. A colorless oily product compound (750 mg, 29.5%) was obtained by column chromatography (petroleum ether: ethyl acetate = 50:1 to 20:1).

In this example, the nuclear magnetic data of Compound 1 is as follows: ¹H NMR (400 Mz, CDCl₃) 4.22-4.11 (m, 8H), 2.62-2.57 (q, *J1* = 8.0 Hz, *J2* = 8.0 Hz, 6H), 2.32-2.28 (t, *J=* 8.0 Hz, 3H), 2.04-1.96 (m, 3H), 1.91-1.85 (m, 2H), 1.81-1.71 (m, 4H), 1.64-1.57 (m, 4H), 1.47-1.42 (m, 2H), 1.31-1.27 (m, 38H), 1.08-1.05 (t, *J =* 12.0 Hz, 6H), 0.91-0.88 (t, *J =* 8.0 Hz, 9H). LCMS: Rt: 1.767 min; MS m/z (ESI): 740.60 [M+H]⁺.
2.

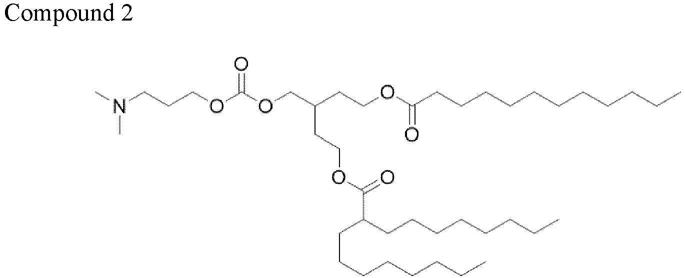

The preparation steps of Compound 2 are as follows:

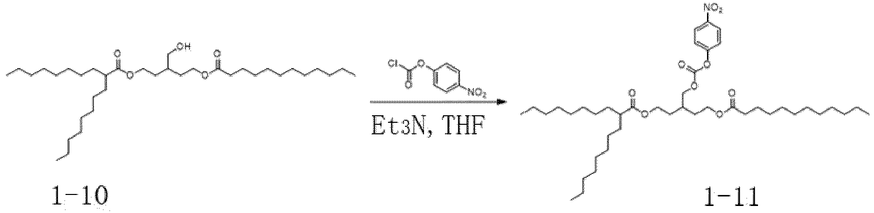

Raw material 1-10 (9.0 g, 15.3 mmol), p-nitrophenyl chloroformate (4.7 g, 23 mmol), triethylamine (3.1 g, 30.6 mmol), and tetrahydrofuran (50 mL) were added in a 100 mL single-necked flask at room temperature for reaction overnight, TLC monitoring showed that most of the raw material was completely reacted. Then, ethyl acetate (200 mL) and water (200 mL) were added for the extraction and liquid separation. The organic phase was then washed with saturated ammonium chloride 2-3 times, dried, and spin-dried. Then, silica gel was added for mixing. A colorless oily product 1-11 (10.68 g, 92%) was obtained by column chromatography (petroleum ether: ethyl acetate = 50:1 to 20:1).

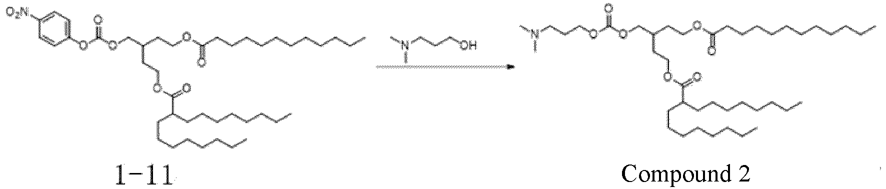

Raw material 1-11 (1.5 g, 2 mmol) was added in a 25 mL single-necked flask at room temperature, and dimethylaminopropanol (0.62 g, 23 mmol) was slowly add under stirring. After reacting for 2 hours, TLC monitoring showed that most of the raw material was completely reacted. Then, ethyl acetate (200 mL) and water (200 mL) were added for the extraction and liquid separation. The organic phase was then washed with saturated ammonium chloride 2-3 times, dried, and spin-dried. Then, silica gel was added for mixing. A yellow oily product was obtained by column chromatography (dichloromethane: methanol = 50:1).

The nuclear magnetic data of Compound 2 is as follows: ¹H NMR (400 Mz, CDCl₃) δ 4.21-4.09 (m,8H), 2.41-2.17 (m,11H), 2.01-1.36(m,13H), 1.24(s,40H), 0.91- 0.83(m,9H). C₄₂H₈₁NO₇ Exact Mass: 711.60, found [M+H]⁺: 712.7.
3.

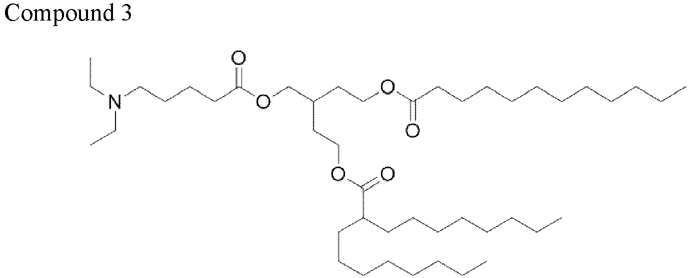

The preparation steps of Compound 3 are as follows:

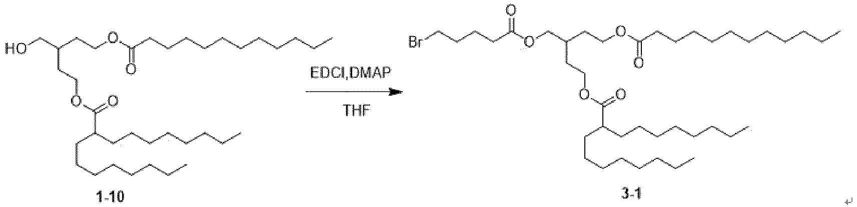

Raw material 1-10 (1.5 g, 2.57 mmol), bromovaleric acid (1.29 g, 3.09 mmol), 1-(3-dimethylaminopropyl)-3-ethylcarbodiimide hydrochloride (12.2 g, 64.0 mmol), 4-dimethylaminopyridine (0.63 g, 5.15 mmol) and tetrahydrofuran (15 mL) were added in a 100 mL single-necked flask at room temperature for reaction overnight. TLC monitoring showed that most of the raw material was completely reacted. Then, ethyl acetate (100 mL) and water (100 mL) were added for the extraction and liquid separation. The organic phase was then washed with saturated ammonium chloride 2-3 times, dried, and spin-dried. Then, silica gel was added for mixing. A colorless oily product 3-1 (1.8 g) was obtained by column chromatography (petroleum ether: ethyl acetate = 50:1 to 20:1).

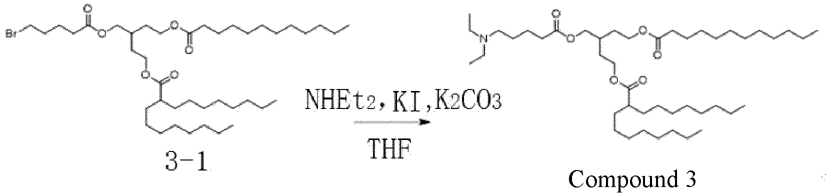

Raw material 3-1 (1.8 g, 2.41 mmol), potassium carbonate (1.59 g, 4.82 mmol), potassium iodide (0.4 g, 2.41 mmol), diethylamine (0.26 g, 3.62 mmol) and anhydrous tetrahydrofuran (15 mL) were added in a 100 mL single-necked flask at room temperature. After reflux reaction for overnight, TLC monitoring showed that most of the raw material was completely reacted. Then, ethyl acetate (100 mL) and water (100 mL) were added for extraction, washing, and liquid separation. The organic phase was then washed with an aqueous ammonium chloride solution for 2-3 times, dried, and spin-dried to obtain a colorless oily crude product. The colorless oily crude product was purified by column chromatography (dichloromethane: methanol = 200:1 to 40:1) to obtain the final product.

The nuclear magnetic data of Compound 3 is as follows: ¹H NMR (400 Mz, CDCl₃) δ 4.13 (m, *J =* 6.8, 1.7Hz,4H), 4.04 (d, *J =* 5.4Hz,2H), 2.66-2.46 (m, 6H), 2.37-2.25 (m, 5H), 1.91(m, *J* = 6.1 Hz,1H), 1.76-1.38 (m,15H), 1.35-1.19 (m, 39H), 1.06 (t, *J =* 7.2 Hz, 6H), 0.87 (t, *J =* 6.7 Hz, 9H). C₄₅H₈₇NO₆ Exact Mass: 737.65, found [M+H]⁺: 738.67.
4.

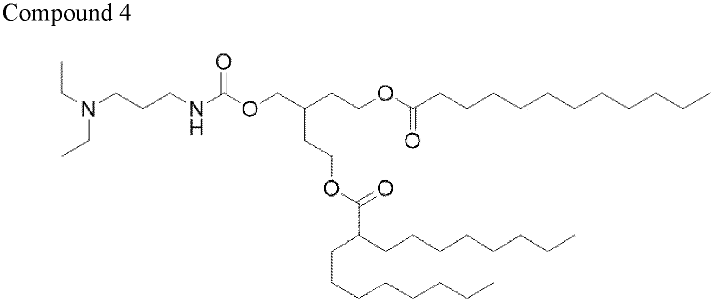

The preparation steps of Compound 4 are as follows:

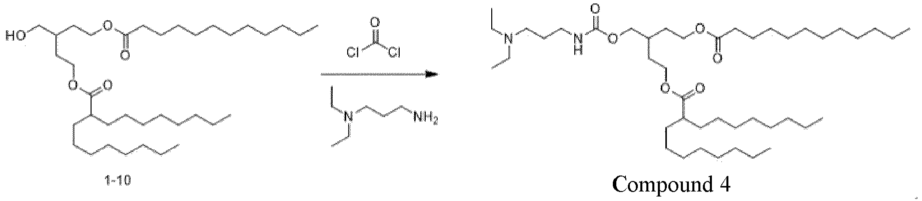

Raw material 1-10 (1.5 g, 2.57 mmol) was added in a 25 mL single-necked flask at room temperature, and solid phosgene (0.28 g, 2.83 mmol) and pyridine (0.31 g, 3.86 mmol) were slowly added to react for 1 hour under stirring, and then diethylaminopropylamine (0.34 g, 2.57 mmol) was added to react for 1 hour. TLC monitoring showed that most of the raw material was completely reacted. Then, ethyl acetate (200 mL) and water (200 mL) were added for the extraction and liquid separation. The organic phase was then washed with saturated ammonium chloride 2-3 times, dried, and spin-dried. Then, silica gel was added for mixing. A yellow oily product Compound 4 (530 mg) was obtained by column chromatography (dichloromethane: methanol = 200:1 to 50:1).

The nuclear magnetic data of Compound 4 is as follows: ¹H NMR (400 Mz, CDCl₃) δ 6.10 (t, *J =* 5.4Hz, 1H), 4.21-3.95 (m, 6H), 3.26 (m, *J =* 6.0Hz, 2H), 2.58 (m, *J =* 7.3Hz, 6H), 2.28 (m, *J =* 6.8Hz, 3H), 1.97-1.83 (m, 1H), 1.79 -1.50 (m, 10H), 1.26 (dd, *J =* 13.3, 4.0 Hz, 42H), 1.07 (t, *J =* 7.1 Hz, 6H), 0.87 (t, *J =* 6.6 Hz, 9H). C₄₄H₈₆N₂O₆ Exact Mass: 738.65, found [M+H]⁺: 739.7.
5.

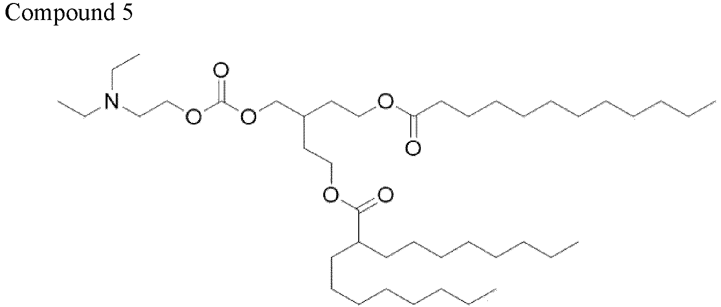

The preparation steps of Compound 5 are as follows:

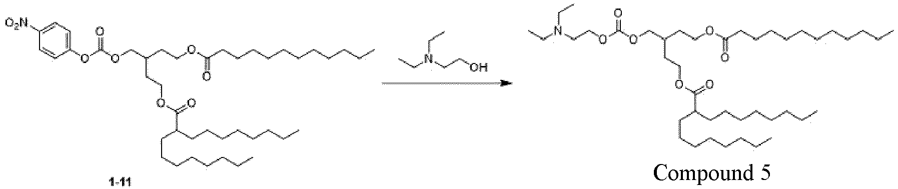

Raw material 1-11 (1.5 g, 2 mmol) was added in a 25 mL single-necked flask at room temperature, and then diethylaminoethanol (0.7 g, 6.02 mmol) was slowly added under stirring. After reacting for 2 hours, TLC monitoring showed that most of the raw material was completely reacted. Then, ethyl acetate (100 mL) and water (100 mL) were added for the extraction and liquid separation. The organic phase was then washed with saturated ammonium chloride 2-3 times, dried, and spin-dried. Then, silica gel was added for mixing. A yellow oily product Compound 5 (550 mg) was obtained by column chromatography (dichloromethane: methanol = 200:1 to 50:1).

The nuclear magnetic data of Compound 5 is as follows: ¹H NMR (400 Mz, CDCl₃) δ 4.25 (t, *J =* 6.2 Hz,2H), 4.12 (dd, *J =* 8.4,5.6Hz,6H), 2.80(t, *J* = 6.2Hz,2H), 2.65 (m, *J* =7.1Hz, 4H), 2.35-2.23 (m, 3H), 1.94 (m, *J =* 6.0 Hz,1H), 1.81-1.50 (m,8H), 1.49-1.16(m, 42H), 1.08(t, *J* = 7.1Hz,6H), 0.93-0.81 (m,9H). C₄₃H₈₃NO₇ Exact Mass: 725.62, found [M+H]⁺: 726.63.
6

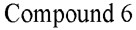

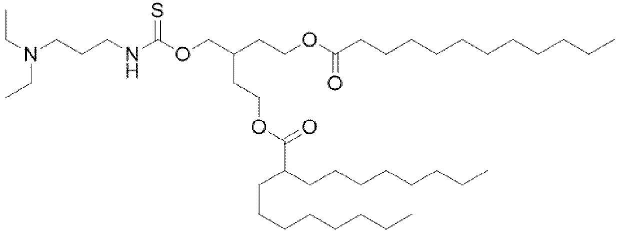

The preparation steps of Compound 6 are as follows:

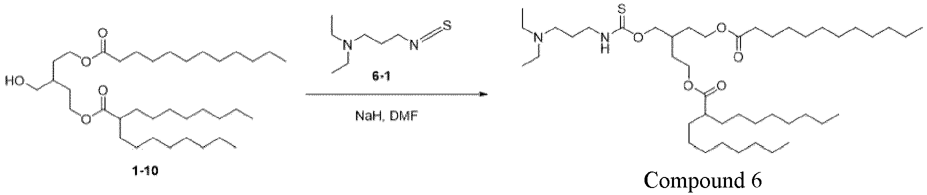

In a 500mL three-necked flask in which a thermometer was inserted, raw material 1-10 (500 mg, 0.86 mmol) and DMF (30 mL) were added respectively to dissolve under ice-water bath conditions. After being cooled to 0-5 °C, and the weighed sodium hydride (60% content 1.1 mmol, 44 mg) was slowly added to the reaction system in batches. After 30 minutes of reaction, raw material 6-1 (163 mg, 0.94 mmol) was slowly added to the above system. After the completion of the dropwise addition, reaction was carried out for 2 hours. TLC color development showed that the raw material was completely reacted. Saturated ammonium chloride solution was slowly added to the reaction solution for quenching, and then ethyl acetate was added for extraction, liquid separation, drying, and spin drying to obtain a light-yellow oily liquid. Then, silica gel was added for mixing. A colorless oily product Compound 6 was obtained by column chromatography.

Herein, the synthesis of raw material 6-1 is based on J. Med. Chem. 2003, 46, 6, 936-953.

The nuclear magnetic data of Compound 6 is as follows: ¹H NMR (400 Mz, CDCl₃) δ 9.28-9.03 (m, 1H), 4.50-4.30 (m, 2H), 4.23-4.07 (m,4H), 3.61 (t, *J* = 6.1 Hz, 1H), 3.39 (m, *J* = 5.5 Hz, 1H), 2.60-2.48 (m,5H), 2.29 (m, *J =* 6.9, 3.3 Hz, 3H), 2.00 (m, *J =* 12.2,6.4 Hz, 1H), 1.82-1.15 (m, 53H), 1.06 (m, *J* = 16.3, 7.1 Hz, 6H), 0.87 (t, J = 6.6 Hz, 9H). C₄₄H₈₆N₂O₅S Exact Mass: 754.63, found [M+H]⁺: 755.69.
7.

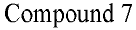

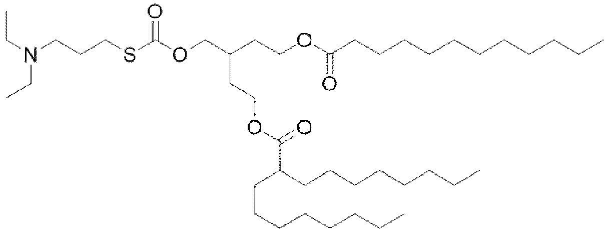

The preparation steps of Compound 7 are as follows:

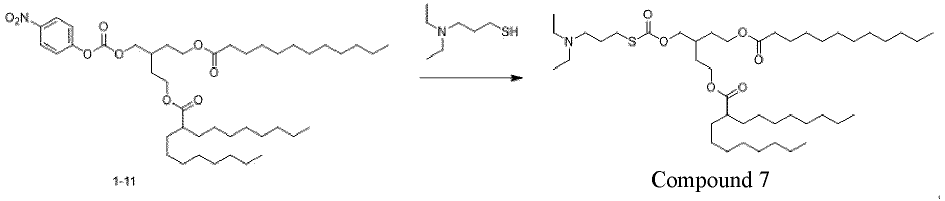

Raw material 1-11 (1.5 g, 2 mmol) was added in a 25 mL single-necked flask at room temperature, and then diethylaminopropylthiol (0.89 g, 6.02 mmol) was slowly added under stirring. After 2 hours of reaction, TLC monitoring showed that most of the raw material was completely reacted. Then, ethyl acetate (100 mL) and water (100 mL) were added for the extraction and liquid separation. The organic phase was then washed with saturated ammonium chloride 2-3 times, dried, and spin-dried. Then, silica gel was added for mixing. A brown oily product Compound 7 (570 mg) was obtained by column chromatography (dichloromethane: methanol = 200:1 to 80:1).

The nuclear magnetic data of Compound 7 is as follows: ¹H NMR (400 Mz, CDCl₃) δ 4.20 (d, *J* = 5.2 Hz, 2H), 4.16-4.09 (m, 4H), 2.90 (t, *J =* 7.1 Hz, 2H), 2.76 - 2.56 (m, 5H), 2.29 (m, *J* = 6.7 Hz, 3H), 1.94 (dd, *J* = 17.7, 11.4 Hz, 3H), 1.75-1.67 (m, 4H), 1.66-1.50 (m, 5H), 1.48-1.38 (m, 2H), 1.36 - 1.18 (m, 40H), 1.11 (s, 6H), 0.87 (t, *J =* 6.7 Hz, 9H).C₄₄H₈₅NO₆S Exact Mass: 755.61, found [M+H]⁺: 756.62.
8.

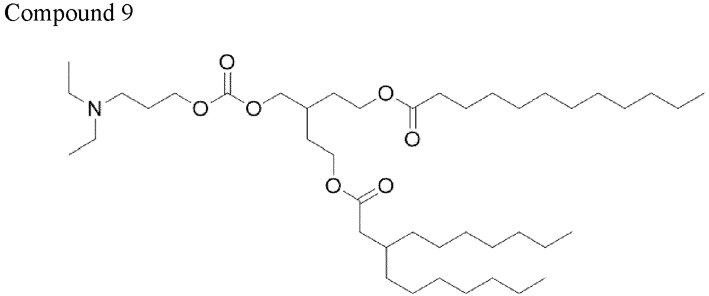

The preparation steps of Compound 9 are as follows:

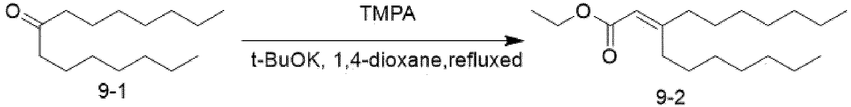

At room temperature, trimethyl phosphonoacetate (11.5 g, 63.2 mmol) and tetrahydrofuran (500 mL) were added in a 500 mL eggplant-shaped bottle, and then potassium tert-butoxide (8.7 g, 77.7 mmol) was gradually added. The mixture was stirred and reacted for 1 hour, and then 9-1 (11 g, 48.6 mmol) was slowly added. After refluxing and stirring for 3 hours, TLC showed that the reaction of the raw material was completely reacted. Then, ethyl acetate (500 mL) and water (500 mL) were added for the extraction and liquid separation. The organic phase was then washed with saturated sodium chloride solution (300 mL) for 2-3 times, dried and spin-dried. Then, silica gel was added for mixing. A light-yellow liquid product 9-2 (8 g) was obtained by column chromatography (petroleum ether: ethyl acetate = 100:1 to 30:1).

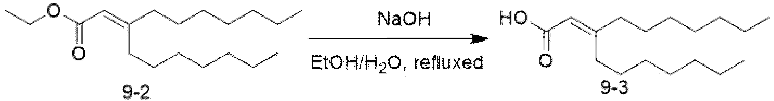

Raw material 9-2 (8g, 111mmol) and anhydrous ethanol (20mL) were added in a 250mL three-necked flask at room temperature, and then 40mL of aqueous sodium hydroxide solution (5.4 g, 134.9mmol) was added. After the addition was completed, the reaction solution was slowly heated to reflux. After reacting for 2h, TLC monitoring showed that the raw material was completely reacted. Then, the ethanol was evaporated, and the pH was adjusted to 2 with 3mol/L hydrochloric acid aqueous solution. After washing the organic phase 3 times with ethyl acetate (50mL), the organic phases were combined and spin-dried. Then, silica gel was added for mixing. A colorless liquid product 9-3 (3.5g) was obtained by column chromatography (dichloromethane: methanol = 50:1 to 20:1).

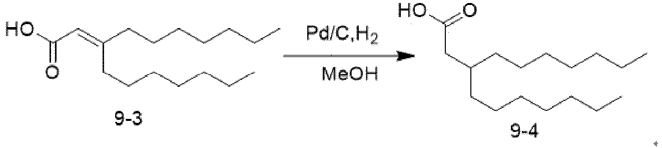

At room temperature, compound 9-3 (3.5 g, 13 mmol) and 40 mL of methanol were added in a 250 mL eggplant-shaped bottle, and then palladium carbon (0.14 g) was added. After replacing with hydrogen, the mixture was stirred and reacted at room temperature for 18 hours. TLC spot plate showed that the raw material was completely reacted. Then, it was filtered, and the organic phase was spin-dried. Silica gel was added for mixing. A colorless liquid product 9-4 (3.3 g) was obtained by column chromatography (petroleum ether: ethyl acetate = 100:1 to 5:1).

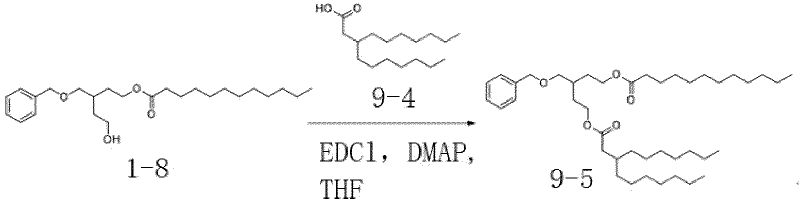

Raw material 1-8 (7.3g, 17.9mmol), compound 9-4 (3.3g, 12.2mmol), 1-(3-dimethylaminopropyl)-3-ethylcarbodiimide hydrochloride (4.1g, 21.5mmol), 4-dimethylaminopyridine (2.63g, 21.5mmol) and dichloromethane (100mL) were added in a 250mL single-necked flask at room temperature for reaction overnight. TLC monitoring showed that most of the raw material was completely reacted. Then, dichloromethane (200mL) and water (200mL) were added for the extraction and liquid separation. The organic phase was then washed with saturated ammonium chloride 2-3 times, dried, and spin-dried. Then, silica gel was added for mixing. A light-yellow oily product 9-5 (7.6g) was obtained by column chromatography (petroleum ether: ethyl acetate = 100:1 to 20:1).

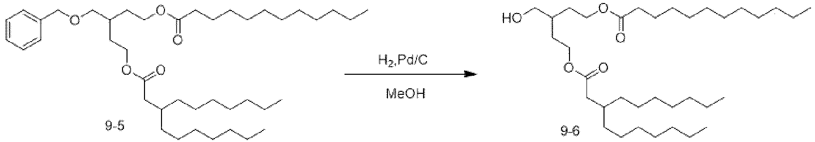

At room temperature, compound 9-5 (7.6 g, 6.4 mmol) and methanol (60 mL) were added in a 250 mL eggplant-shaped bottle, and then palladium carbon (760 mg) was added. The air in the bottle was replaced with hydrogen. After stirring at room temperature for reaction overnight, TLC showed that the raw material was completely reacted. The palladium carbon was removed by suction filtration. The organic phase was then dried and spin-dried. Silica gel was added for mixing. A colorless liquid product 9-6 (5 g) was obtained by column chromatography (petroleum ether: ethyl acetate = 30:1 to 5:1).

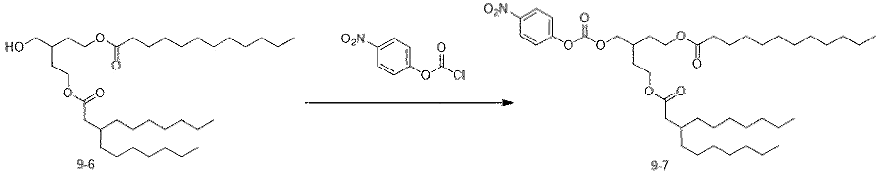

At room temperature, compound 9-6 (5 g, 8.8 mmol) and tetrahydrofuran (37 mL) were added in a 250 mL sealed tube. p-Nitrophenyl chloroformate (2.7 g, 13.2 mmol) was added and stirred for 10 minutes, and then triethylamine (1.8 g, 17.6 mmol) was added and reacted at room temperature for 15 minutes, and then the temperature was raised. The reaction was refluxed for 3 hours, and the TLC spot plate showed that the raw material was completely reacted. Then, water was added to quench the reaction. Then, ethyl acetate (300 mL) and water (300 mL) were added for the extraction and liquid separation. The organic phase was then washed with saturated sodium chloride solution (300 mL) for 2-3 times, dried and spin-dried. Silica gel was added for mixing. A colorless liquid product 9-7 (4.7 g) was obtained by column chromatography (petroleum ether: ethyl acetate = 100:1 to 10:1).

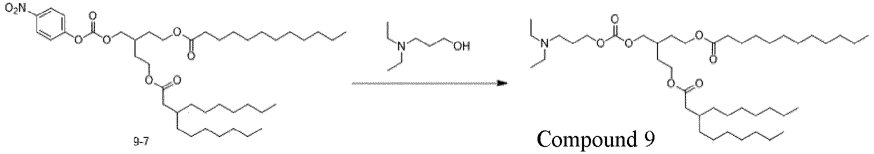

At room temperature, compound 9-7 (2.5 g, 3.4 mmol) was added in a 50 mL eggplant-shaped bottle, and then 3-Diethylaminopropanol (2.2 g, 17 mmol) was gradually and slowly added, and the mixture was stirred and reacted for 50 minutes. TLC spot plate showed that the raw material was completely reacted. Then, water was added to quench the reaction. Then, ethyl acetate (200 mL) and water (200 mL) were added for the extraction and liquid separation. The organic phase was then washed with saturated sodium chloride solution (200 mL) 2-3 times, dried and spin-dried. Silica gel was added for mixing. A crude product was obtained by column chromatography (dichloromethane: methanol = 300:1 to 80:1). Further, the crude product was purified to obtain a yellow liquid Compound 9 (639 mg).

The nuclear magnetic data of Compound 9 is as follows: ¹H NMR (400 Mz, CDCl₃) δ 4.21 (t, *J* = 6.4 Hz,2H), 4.18-4.10 (m,5H), 2.65 (s,5H), 2.30 (t, *J* = 7.6 Hz, 2H), 2.24 (d, J = 6.9 Hz, 2H), 1.96 (dd, *J* = 13.3, 7.1 Hz, 3H), 1.80 (s, 2H), 1.80-1.67 (m, 4H), 1.62 (q, *J* = 7.3 Hz, 2H), 1.28 (d, *J =* 5.6 Hz, 39H), 1.11 (t, *J =* 7.3 Hz, 6H), 0.90 (t, J = 6.7 Hz, 9H). C₄₃H₈₃NO₇ Exact Mass:725.62, found [M+H]⁺: 726.67.
9.

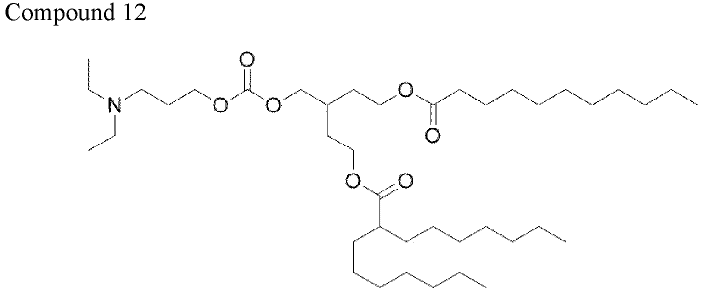

The preparation steps of Compound 12 are as follows:

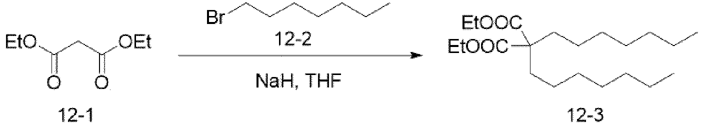

Compound 12-1 (40.0 g, 249.74 mmol) was added in a 2000 mL three-necked flask and dissolved in tetrahydrofuran (800.0 mL), and then sodium hydride (39.96 g, 998.94 mmol) was slowly added in a water bath and stirred for 5 minutes. Compound 12-2 (178.91 g, 998.94 mmol) was added thereto. The mixture was refluxed and stirred at 80 °C for reaction overnight. The reaction was monitored by TLC and LCMS. LCMS detected the target molecular weight, and in TLC (petroleum ether: ethyl acetate = 20:1), iodine coloration showed that the raw material was completely reacted, and a new main spot was generated. The reaction was stopped. Aqueous solution was added for washing, and the aqueous solution was extracted with ethyl acetate three times, and then the organic phase was collected. The organic phase was dried with sodium sulfate, and concentrated under reduced pressure. Then, silica gel was added for mixing. A colorless oily liquid compound 12-3 (39 g) was obtained by column chromatography (first pure petroleum ether column chromatography was used, and then 20:1 of petroleum ether and ethyl acetate column chromatography was used). The molecular weight of the compound was 356.55. LC-MS (m/z) [M+H]=357, Rt=3.492 min.

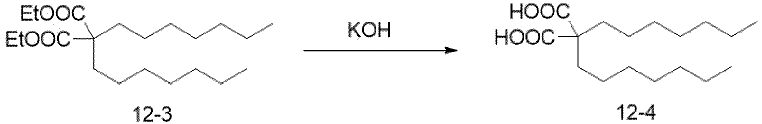

Compound 12-3 (39 g, 109.38 mmol) was added in a 500 mL single-necked flask and dissolved in ethanol (39.0 mL). Potassium hydroxide (61.37 g, 1093.82 mmol) was dissolved in H₂O (109.0 mL), and stirred until the solution became clear, and then the solution was added to the reaction solution containing compound 12-3, and stirred at 95 °C for reaction overnight. The reaction was monitored by TLC. LCMS detected the target molecular weight, and in TLC (petroleum ether: ethyl acetate = 20:1), iodine coloration showed that the raw material was completely reacted, and a new main spot was generated. The reaction was stopped. The reaction solution was concentrated under reduced pressure to spin off most of the ethanol, and the pH was adjusted to 2-3 with 6 mol/L hydrochloric acid solution. The resulting product was extracted with ethyl acetate and water, and then liquid separation treatment was performed. The organic phase was collected, dried, and concentrated under reduced pressure to obtain a white solid compound 12-4 (31.11 g). The molecular weight of the compound is 300.44. LC-MS (m/z) [M-H] = 299, Rt = 2.791min.

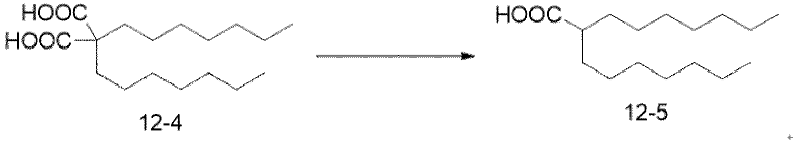

Compound 12-4 (31.11 g, 103.55 mmol) was added in a 500 mL single-necked flask, and then a condenser pipe was connected to the flask and a balloon was inserted therein. The temperature was raised to 170 °C, and the reaction was refluxed and stirred for 5 hours. The balloon was observed, until the balloon was no longer expanded. The gas release was stopped, and then the reaction was stopped. The reaction was monitored by nuclear magnetic resonance. A brown liquid compound was obtained, and the brown liquid compound was concentrated under reduced pressure. Then, silica gel was added for mixing. A yellow viscous solid compound 12-5 (25.5 g) was obtained by column chromatography (first pure petroleum ether column chromatography was used, and then 10:1 of petroleum ether and ethyl acetate column chromatography was used).

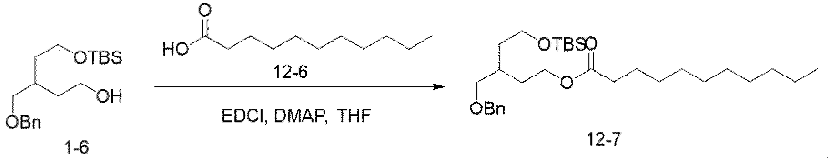

Compound 12-6 (4.40 g, 23.63 mmol) was added in a 250 mL single-necked flask and dissolved in tetrahydrofuran (80.0 mL). 1-(3-dimethylaminopropyl)-3-ethylcarbodiimide hydrochloride (6.79 g, 35.44 mmol) was added thereto and stirred at room temperature for 0.5 hours. 4-dimethylaminopyridine (5.77 g, 47.26 mmol) was added and stirred at room temperature for 15 minutes. Then, compound 9-6 (8 g, 23.63 mmol) was added and stirred at room temperature for reaction overnight. The reaction was monitored by TLC. In TLC (petroleum ether: ethyl acetate = 20:1), iodine coloration showed that the raw material was completely reacted, and a new main spot was generated. The reaction was stopped. The resulting product was extracted with ethyl acetate and saturated ammonium chloride solution, and then liquid separation treatment was performed. The organic phase was collected, dried, and spin-dried. A yellow oily liquid compound 12-7 (9.27 g) was obtained by column chromatography (petroleum ether: ethyl acetate = 20:1).

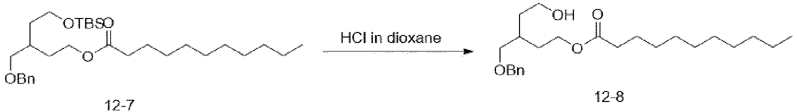

Compound 12-7 (9.27 g, 18.29 mmol) was added in a 100 mL single-necked flask, and dissolved in dioxane (14.0 mL), and then the concentrated hydrochloric acid (19 mL) was added thereto and stirred at room temperature for 2 hours. The reaction was monitored by TLC. In TLC (petroleum ether: ethyl acetate = 5:1), iodine coloration showed that most of the raw material was completely reacted, and a small part of the raw material was remained, and a new main spot was generated. The reaction was stopped. The resulting product was extracted with ethyl acetate and water, and then liquid separation treatment was performed. The organic phase was collected, dried, and spin-dried. A yellow oily liquid compound 12-8 (5.53 g) was obtained by column chromatography (petroleum ether: ethyl acetate = 100:1-5:1).

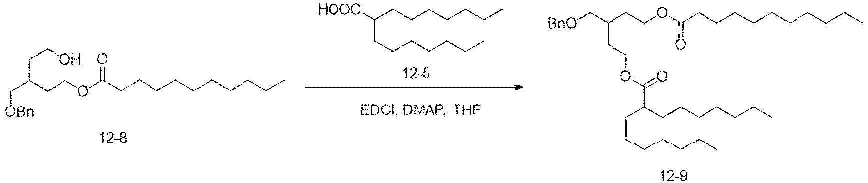

Compound 12-8 (3.61 g, 14.09 mmol) was added in a 250 mL single-necked flask and dissolved in tetrahydrofuran (50.0 mL), and then 1-(3-dimethylaminopropyl)-3-ethylcarbodiimide hydrochloride (4.05 g, 21.13 mmol) was added and stirred at room temperature for 0.5 hours. Then, 4-dimethylaminopyridine (3.44 g, 28.17 mmol) was added and stirred at room temperature for 15 minutes, and further compound 12-5 (5.53 g, 14.09 mmol) was added and stirred at room temperature for reaction overnight. The reaction was monitored by TLC. In TLC (petroleum ether: ethyl acetate = 20:1), iodine coloration showed that the raw material was completely reacted, and a new main spot was generated. The reaction was stopped. The resulting product was extracted with saturated ammonium chloride solution and ethyl acetate, and then liquid separation treatment was performed. The organic phase was collected, dried, and concentrated under reduced pressure. A yellow oily liquid compound 12-9 (6.64 g) was obtained by column chromatography (petroleum ether: ethyl acetate = 20:1).

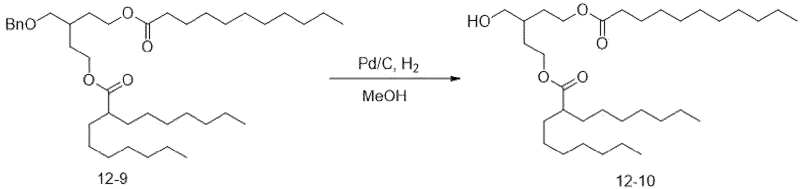

Compound 12-9 (6.64 g, 10.76 mmol) was added in a 250 mL single-necked flask and dissolved in methanol (50.0 mL), and then palladium-carbon (0.9 g) was added. The flask was replaced with hydrogen for 5 times. The mixture was stirred at room temperature under hydrogen for reaction overnight. The reaction was monitored by TLC. In TLC (petroleum ether: ethyl acetate = 5:1), iodine coloration showed that the raw material was completely reacted, and a new main spot was generated. The reaction was stopped. The palladium-carbon was filtered out by suction filtration using silica gel powder, and then the palladium-carbon remaining in the reaction solution was filtered out with a filter head. The resulting product was concentrated under reduced pressure to obtain a colorless oily liquid compound 12-10 (5.16 g).

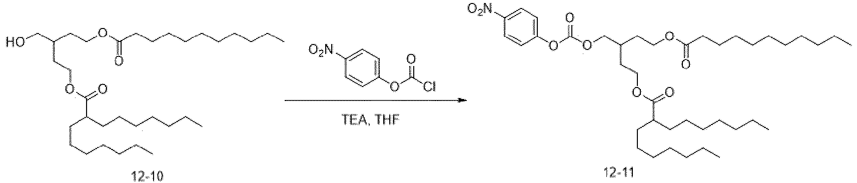

Compound 12-10 (5.16 g, 9.54 mmol) was added in a 250 mL sealed tube and dissolved in tetrahydrofuran (40.0 mL), and then p-nitrophenyl chloroformate (2.88 g, 14.31 mmol) was added thereto. The mixture was stirred at room temperature for 5 minutes until the mixture became clear. Triethylamine (1.93 g, 19.08 mmol) was added at room temperature and stirred at room temperature for 5 minutes. The lid was covered. The mixture was stirred at 70°C for reaction overnight. The reaction was monitored by TLC. In TLC (petroleum ether: ethyl acetate = 10:1), iodine coloration showed that the raw material was completely reacted, and a new main spot was generated, and ultraviolet light was generated. The reaction was stopped. Aqueous solution, ethyl acetate and saturated ammonium chloride solution were added in sequence for washing, and the resulting product was extracted with ethyl acetate twice. The organic phase was collected, dried with sodium sulfate, and concentrated under reduced pressure. Then, silica gel was added for mixing. A yellow liquid compound 12-11 (3.04 g) was obtained by column chromatography (first pure petroleum ether column chromatography was used, and then 40:1 of petroleum ether and ethyl acetate column chromatography was used).

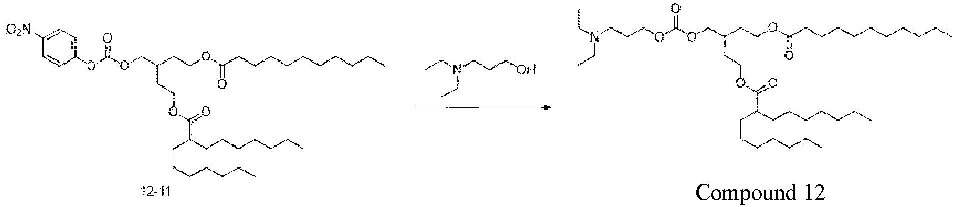

Compound 12-11 (3.04 g, 4.31 mmol) was added in a 50 mL single-necked flask, and then 3-(diethylamino)propyl-1-ol (5.65 g, 43.06 mmol) was added. The mixture was stirred at room temperature for reaction overnight. The reaction was monitored by TLC and LCMS. In TLC (dichloromethane: methanol = 10:1), iodine coloration showed that the raw material was completely reacted, and a new main spot was generated. The reaction was stopped. LCMS detected the target molecular weight. Saturated ammonium chloride solution was added for washing, and the resulting product was extracted with ethyl acetate 3 times. The organic phase was collected, dried with sodium sulfate, and concentrated under reduced pressure. Then, silica gel was added for mixing. 1.7 g of a yellow liquid was obtained by column chromatography (dichloromethane: methanol = 70:1), and further the yellow liquid was purified to obtain a yellow oily liquid compound 12 (632 mg). The molecular weight of compound 12 was 698.08. LC-MS (m/z) [M+H] = 698, Rt = 5.76 min.

The nuclear magnetic data of compound 12 is as follows: ¹H NMR (400 Mz, CDCl₃) δ 4.20 (t, *J=* 6.6 Hz, 2H), 4.18-4.09(m,6H), 2.54 (q, *J =* 7.1 Hz, 6H), 2.36-2.26 (m, 3H), 1.97(p, *J* = 5.9 Hz, 1H), 1.84 (m, 2H), 1.80-1.68 (m, 4H), 1.67-1.52 (m,4H), 1.50-1.38 (m,2H), 1.35-1.21 (m,34H), 1.04 (t, *J =* 7.1Hz, 6H), 0.90(m,9H). C₄₁H₇₉NO₇ Exact Mass: 697.59, found [M+H]⁺: 698.60.
10.

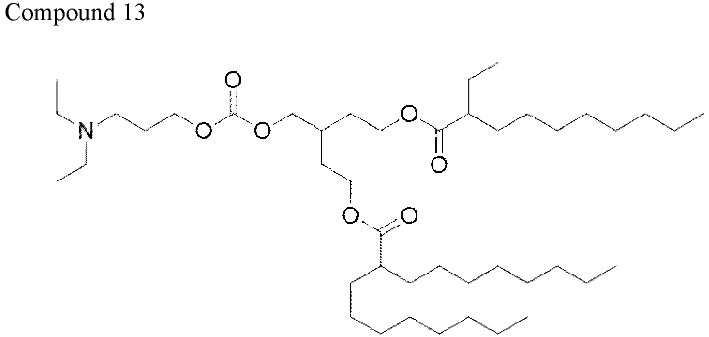

The preparation steps of Compound 13 are as follows:

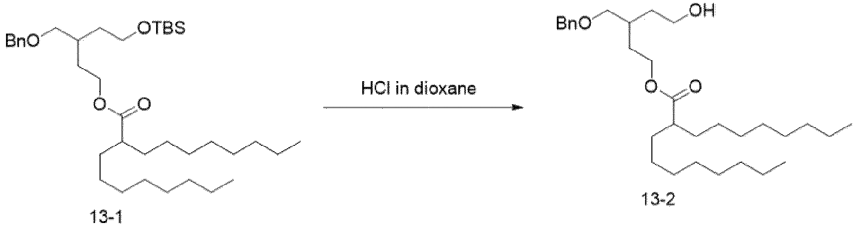

Compound 13-1 (8.66 g, 14.31 mmol) was added in a 100 mL single-necked flask, and dissolved in dioxane (10.0 mL), and then concentrated hydrochloric acid (18 mL) was added and stirred at room temperature for 2 h. The reaction was monitored by TLC. In TLC (petroleum ether: ethyl acetate = 5:1), iodine coloration shows that most of the raw material was completely reacted, and a new main spot was generated. The reaction was stopped. The resulting product was extracted with ethyl acetate and water, and then liquid separation treatment was performed. The organic phase was collected, dried, and spin-dried. A yellow oily liquid compound 13-2 (4.98 g) was obtained by column chromatography (petroleum ether: ethyl acetate = 100:1-5:1).

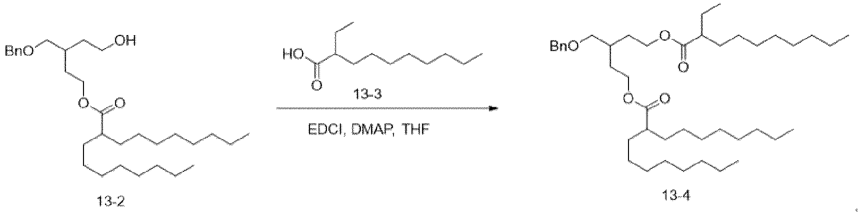

Compound 13-2 (2.44 g, 12.18 mmol) was added in a 250 mL single-necked flask and dissolved in tetrahydrofuran (50.0 mL), and then 1-(3-dimethylaminopropyl)-3-ethylcarbodiimide hydrochloride (2.92 g, 15.22 mmol) was added and stirred at room temperature for 0.5 hours. Then, 4-dimethylaminopyridine (2.48 g, 20.29 mmol) was added and stirred at room temperature for 15 minutes, and further compound 13-3 (4.98 g, 10.147 mmol) was added and stirred at room temperature for reaction overnight. The reaction was monitored by TLC. In TLC (petroleum ether: ethyl acetate = 20:1), iodine coloration showed that the raw material was completely reacted, and a new main spot was generated. The reaction was stopped. The resulting product was extracted with saturated ammonium chloride solution and ethyl acetate, and then liquid separation treatment was performed. The organic phase was collected, dried, and concentrated under reduced pressure. A yellow oily liquid compound 13-4 (6.18 g) was obtained by column chromatography (first, pure petroleum ether column chromatography was used, and then with 20:1 of petroleum ether and ethyl acetate column chromatography was used).

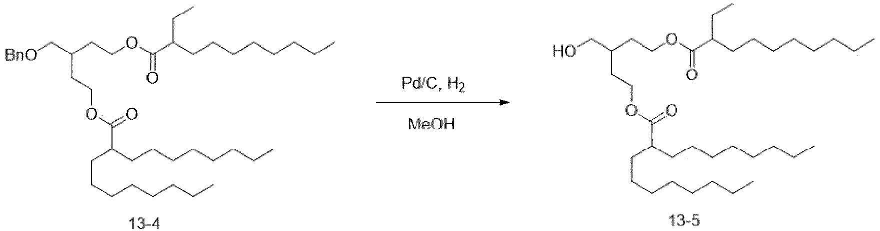

Compound 13-4 (6.18 g, 9.18 mmol) was added in a 500 mL single-necked flask and dissolved in methanol (60.0 mL), and then palladium-carbon (0.90 g) was added. The flask was replaced with hydrogen for 5 times. The mixture was stirred at room temperature under hydrogen for reaction overnight. The reaction was monitored by TLC. In TLC (petroleum ether: ethyl acetate = 5:1), iodine coloration showed that the raw material was completely reacted, and a new main spot was generated. The reaction was stopped. The palladium-carbon was filtered out by suction filtration using silica gel powder, and then the palladium-carbon remaining in the reaction solution was filtered out with a filter head. The resulting product was concentrated under reduced pressure to obtain a colorless oily liquid compound 13-5 (4.97 g).

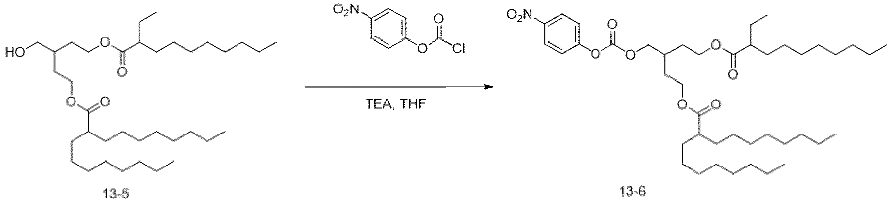

Compound 13-5 (4.97 g, 8.53 mmol) was added in a 250 mL sealed tube and dissolved in tetrahydrofuran (40.0 mL), and then p-nitrophenyl chloroformate (2.58 g, 12.79 mmol) was added. The mixture was stirred at room temperature for 5 minutes until the mixture became clear. Triethylamine (2.370 mL, 17.05 mmol) was added at room temperature and stirred at room temperature for 5 minutes. The lid was covered. The mixture was stirred at 70°C for reaction overnight. The reaction was monitored by TLC. In TLC (petroleum ether: ethyl acetate = 10:1), iodine coloration showed that the raw material was completely reacted, and a new main spot was generated, and ultraviolet light was generated. The reaction was stopped. Aqueous solution, ethyl acetate and saturated ammonium chloride solution were added in sequence for washing, and the resulting product was extracted with ethyl acetate twice. The organic phase was collected, dried with sodium sulfate, and concentrated under reduced pressure. Then, silica gel was added for mixing. A yellow liquid compound 13-6 (4.08 g) was obtained by column chromatography (first, pure petroleum ether column chromatography was used, and then 40:1 of petroleum ether and ethyl acetate column chromatography was used).

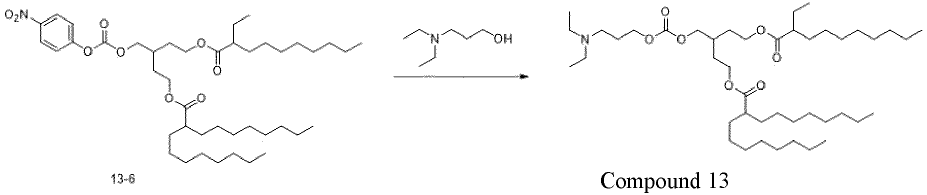

Compound 13-6 (4.08 g, 5.45 mmol) was added in a 50 mL single-necked flask, and then 3-diethylaminopropanol (7.16 g, 54.54 mmol) was added and stirred at room temperature for reaction overnight. The reaction was monitored by TLC and LCMS. In TLC (dichloromethane: methanol = 10:1), iodine coloration showed that the raw material was completely reacted, and a new main spot was generated. The reaction was stopped. LCMS detected the target molecular weight. Saturated ammonium chloride solution was added for washing, and the resulting product was extracted with ethyl acetate 3 times. The organic phase was collected, dried with sodium sulfate, and concentrated under reduced pressure. Then, silica gel was added for mixing. 2.1 g of a yellow liquid was obtained by column chromatography (dichloromethane: methanol = 70:1), and further prepared to obtain a yellow oily liquid Compound 13 (716 mg). The molecular weight of Compound 13 was 740.16. LC-MS (m/z) [M+H] = 740, Rt = 5.91 min.

The nuclear magnetic data of Compound 13 is as follows: ¹H NMR (400 Mz, CDCl₃) δ 4.20 (t, *J=* 6.5 Hz, 2H), 4.18-4.09 (m,6H), 2.61 (q, *J =* 7.1 Hz,6H), 2.36-2.21(m,2H), 2.02-1.84 (m,3H), 1.83-1.67 (m,4H), 1.67-1.54 (m,4H), 1.52-1.37 (m,4H),1.26 (s,36H),1.08 (t, *J=* 7.2 Hz, 6H), 0.89 (m,12H). C₄₄H₈₅NO₇ Exact Mass:739.63, found [M+H]⁺: 740.31.
11.

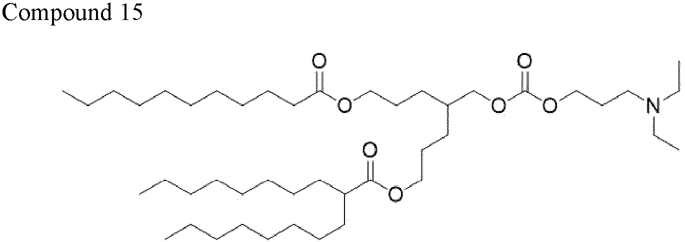

The preparation method of Compound 15 is as follows:

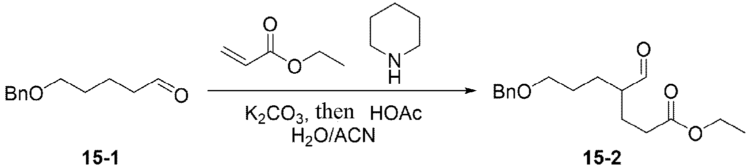

Under ice-water bath conditions, raw material piperidine (1.32g, 15.6mmol) and anhydrous potassium carbonate (0.47g, 3.4mmol) were added in a 100mL flask in which a thermometer was inserted; After mixing evenly, raw material 15-1 (2g, 10.4mmol) was slowly dripped into the reaction system, and reacted at 0°C for 2 hours. The temperature of the reaction solution was slowly raised to room temperature, and the reaction was continued for 36 hours. After the reaction was completed, the obtained mixture was filtered, and the filter cake was washed with ether (30mL) 3 times. The filtrate was combined and spin-dried. The obtained mixture was dissolved in acetonitrile (20mL), and ethyl acrylate (1.56g, 15.6mmol) was dripped into the reaction system under ice-water bath conditions. The temperature was raised to room temperature after the dropwise addition was completed. The reaction was refluxed overnight. Aqueous acetic acid solution (0.8g of glacial acetic acid was dissolved in 5mL of water) was added in the reaction system, and then the reaction was continued to heat and reflux for 2 hours. The reaction system was cooled to room temperature. The aqueous phase was adjusted to be saturated with sodium chloride solid, and extracted three times with diethyl ether (20 mL). The organic phase was dried and spin-dried. Then, silica gel was added for mixing. The product 15-2 was obtained by column chromatography.

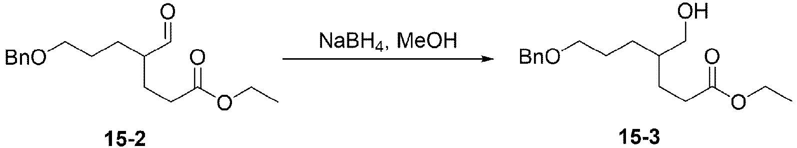

Under ice-water bath conditions, raw material 15-2 (2 g, 6.8 mol) and methanol (50 mL, used to dissolve raw material 15-2) were added respectively in a 100 mL flask in which a thermometer was inserted. After mixing evenly, sodium borohydride solid (310 mg, 8.2 mmol) was slowly and evenly added in the reaction system. The reaction solution was slowly raised to room temperature. After reacting for 4 hours, the reaction was monitored by TLC until the reaction was complete. Water (10 mL) was slowly added in the reaction solution for quenching, and then ethyl acetate (50 mL) was added for dilution. Then, extraction and separation were performed. The organic phase was washed once with saturated sodium chloride solution (25 mL), dried, and spin-dried. The obtained crude product was used directly in the next step without further purification.

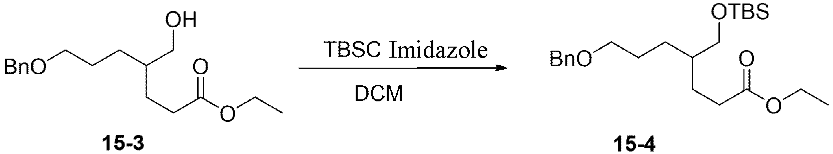

At room temperature, the crude product obtained in the previous step and imidazole (926 mg, 13.6 mmol) were dissolved in dichloromethane (20 mL), and t-butyldimethylchlorosilane (1.54 g, 10.2 mmol) was added in the reaction system in batches. After the addition was completed, the reaction was allowed to proceed overnight. The reaction was monitored by TLC until the reaction was complete. Water (20 mL) was added in the reaction system for the extraction and liquid separation. The aqueous phase was extracted once with dichloromethane (40 mL). The organic phases were combined and washed once with saturated sodium bicarbonate solution (30 mL) and once with saturated sodium chloride solution (30 mL). The organic phase was dried and spin-dried. For the obtained mixture, silica gel was added for mixing. The product 15-4 was obtained by column chromatography.

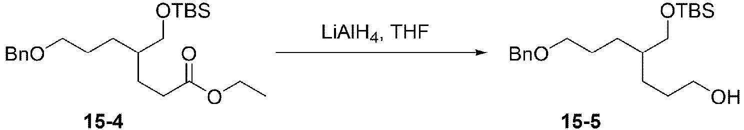

Under ice-water bath conditions, raw material 15-4 (1 g, 2.4 mmol) and anhydrous tetrahydrofuran (30 mL) were added in a 100 mL three-necked flask, and then lithium aluminum tetrahydrogen solid (102 mg, 2.64 mmol) was carefully added in batches. After the addition was completed, the temperature of the reaction solution was slowly raised to room temperature. After reacting for 2 hours, TLC monitoring showed that the raw material was completely reacted. Then, ethyl acetate (20 mL) was added in the reaction solution for dilution, and then water (2 mL), 15% NaOH aqueous solution (2 mL) and water (5 mL) were slowly added for quenching. The reaction solution was filtered by suction. The filter cake was washed with ethyl acetate (30 mL) 3 times. The organic phases were combined, dried, and spin-dried. Silica gel was added for mixing. The product 15-5 was obtained by column chromatography.

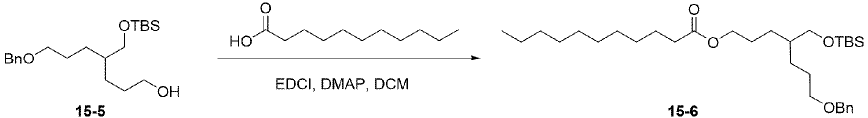

Raw material 15-5 (1.0 g, 2.7 mmol), undecanoic acid (610 mg, 3.3 mmol), 1-(3-dimethylaminopropyl)-3-ethylcarbodiimide hydrochloride (630 mg, 3.3 mmol), 4-dimethylaminopyridine (402 mg, 3.3 mmol) and dichloromethane (20 mL) were added in a 250 mL single-necked flask at room temperature to react; After reacting overnight, the reaction was monitored by TLC until the raw material was completely reacted. Then, ethyl acetate (50 mL) and water (50 mL) were added in the reaction solution for the extraction and liquid separation. The organic phase was washed once with saturated sodium chloride (30 mL), dried, and spin-dried. Then, silica gel was added for mixing. The product 15-6 was obtained by column chromatography.

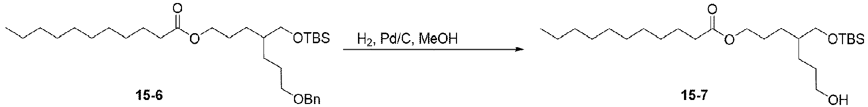

Raw material 15-6 (1.0 g, 1.8 mmol), methanol (20 mL), and palladium carbon containing water (5% palladium content, 100 mg, 10 wt%) were added in a 50 mL single-necked flask at room temperature. The atmosphere in the flask was replaced with nitrogen three times. Then, a hydrogen-filled balloon was attached. After being replaced with hydrogen for 3 times, the reaction was allowed to proceed overnight. The reaction was monitored by TLC until the raw material was completely reacted. Then, diatomaceous earth was used for suction filtration to remove palladium carbon. The filtrate was spin-dried to obtain a crude product. The crude product was directly used in subsequent reactions without further purification.

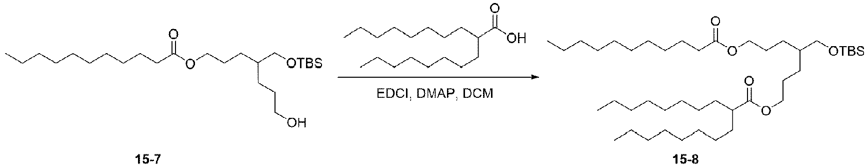

Raw material 15-7 (1.0 g, 2.7 mmol), 2-octyl decanoic acid (767 mg, 3.3 mmol), 1-(3-dimethylaminopropyl)-3-ethylcarbodiimide hydrochloride (518 mg, 3.3 mmol), 4-dimethylaminopyridine (329 mg, 3.3 mmol) and dichloromethane (50 mL) were added in a 100 mL single-necked flask at room temperature to react. After the reaction was allowed to proceed overnight, the reaction was monitored by TLC until the raw material was completely reacted. Then, ethyl acetate (50 mL) and water (50 mL) were added in the reaction system for the extraction and liquid separation. The organic phase was washed once with saturated sodium chloride (30 mL), dried, and then spin-dried. Then, silica gel was added for mixing. The product 15-8 was obtained by column chromatography.

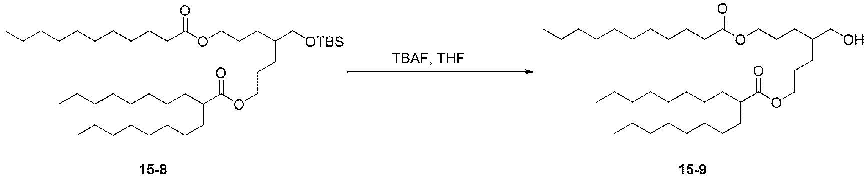

Raw material 15-8 (1.0 g, 1.4 mmol) and tetrahydrofuran (20 mL, used to dissolve raw material 15-8) were added in a 50 mL single-necked flask at room temperature; After mixing evenly, tetrabutylammonium fluoride in tetrahydrofuran solution (1 mol/L, 2.1 mL) was dropwise added thereto. After the dropwise addition was complete, the reaction was continued at room temperature for 2 hours. The reaction was monitored by TLC until the raw material was completely reacted. Water (50 mL) was slowly added to the reaction system, and most of the organic phase was removed under reduced pressure. Ethyl acetate (50 mL) was added for the extraction and liquid separation. The organic phase was washed once with saturated sodium chloride solution (20 mL), dried, and spin-dried. Then, silica gel was added for mixing. The product 15-9 was obtained by column chromatography.

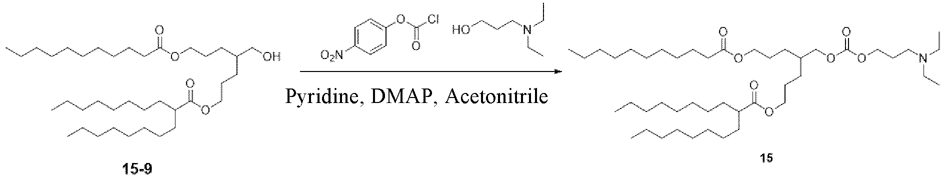

Raw material 15-9 (1.0 g, 1.67 mmol), pyridine (253 mg, 3.2 mmol), 4-dimethylaminopyridine (39 mg, 0.32 mmol), and acetonitrile (20 mL) were added in a 100 mL single-necked flask at room temperature, and fully dissolved, and then p-nitrophenyl chloroformate (482 mg, 2.4 mmol) was slowly added thereto. After reacting for 2 hours, the reaction was monitored by TLC until a new fluorescent spot was generated. Then, 3-dimethylamino-1-propanol (628 mg, 4.8 mmol) was added in the reaction system for reaction overnight. LCMS monitoring showed that the reaction was completed. Ethyl acetate (50 mL) and water (50 mL) were added in the reaction solution for the extraction and liquid separation. The organic phase was washed once with saturated sodium chloride (30 mL), dried, and then spin-dried. Then, silica gel was added for mixing. The final product Compound 15 was obtained by column chromatography.

The nuclear magnetic data of Compound 15 is as follows: ¹H NMR (400 Mz, CDCl₃) δ 4.19 (t, *J =* 6.4 Hz,2H), 4.11-4.00 (m,6H), 2.64(s,5H), 2.35-2.24 (m,3H),1.92(s,2H), 1.75-1.51 (m,10H), 1.41(m, *J*=15.1,7.1,3.7Hz, 6H), 1.34-1.17(m, 38H), 1.10(t, *J =* 7.2Hz,6H), 0.87(m, *J*=6.9,1.4Hz,9H). C₄₅H₈₇NO₇ Exact Mass:753.65, found [M+H]⁺: 754.72.
12.

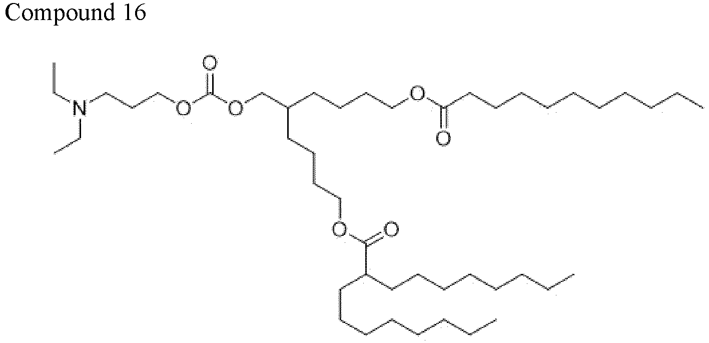

The preparation process of Compound 16 is as follows:

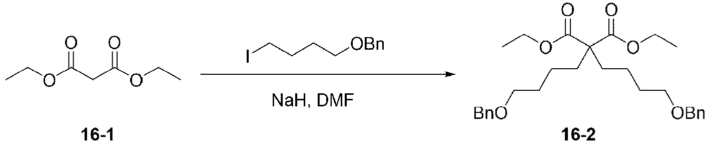

Under ice-water bath conditions, sodium hydride (1.5g, 37.6mmol, w/w 60%) was carefully added into N,N-dimethylformamide (50mL) solution in batches in a 250mL flask in which a thermometer was inserted. After mixing evenly, raw material 16-1 (2 g, 12.5 mmol) in N,N-dimethylformamide (20 mL) solution was slowly dropped into the reaction system. The reaction was carried out for 30 minutes until bubbles no longer emerged. 4-benzyloxyiodobutane (8g, 27.6mmol) in N,N-dimethylformamide (40mL) solution was slowly added into the reaction system, and the reaction was continued for 2 hours. The reaction was monitored by TLC until the raw materials was completely reacted. If the reaction is not complete, the temperature of the system can be slowly raised to 50°C, and the reaction is monitored by TLC until the raw material is reacted complete. Under ice-water bath conditions, saturated ammonium chloride solution (10 mL) was carefully added into the reaction system for quenching, and then water (200 mL) was added to dilute. Ethyl acetate (200 mL) was used for the extraction and liquid separation. The organic phase was washed once with a saturated sodium chloride solution (100 mL), dried, and spin-dried. Then, silica gel was added for mixing. The product 16-2 was obtained by column chromatography.

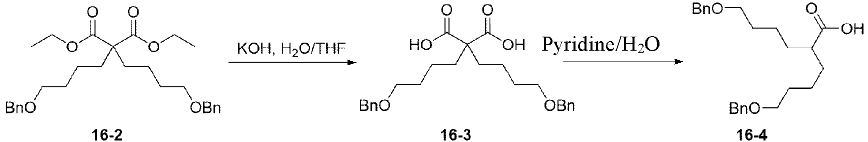

### Reaction step 1:

Raw material 16-22 (2g, 4.3mmol), potassium hydroxide (1.15g, 20.6mmol), water (10mL), and tetrahydrofuran (20mL) were added in a 100mL flask at room temperature; After mixing evenly, the temperature was raised to 70 °C to react for 10 hours. The reaction was monitored by TLC until the raw material was completely reacted. The temperature of the reaction was lowered to room temperature, and the pH value was adjusted to about 6 using 1mol/L hydrochloric acid solution. The organic phase was removed from the system under reduced pressure. Water was added to dilute to 50mL. Ethyl acetate (50mL) was used for the extraction and liquid separation. The organic phase was dried and spin-dried to obtain a crude product. The crude product was used directly for the next step of synthesis without further purification.

### Reaction step 2:

The crude product obtained in the previous step and a mixed solvent of water/pyridine (v/v = 6/1, 50 mL) were added respectively in a 100 mL flask under room temperature conditions. After mixing evenly, the temperature was raised to reflux, and the reaction was continued for 48 hours and cooled. The reaction was monitored by TLC until the raw material was completely reacted; The solvent was removed under vacuum. Water (30 mL) was used to dilute, and the pH value was adjusted to about 6 with 1 mol/L hydrochloric acid solution. Ethyl acetate (50 mL) was used for the extraction and liquid separation. The organic phase was dried and spin-dried. Then, silica gel was added for mixing. The product 16-4 was obtained by column chromatography.

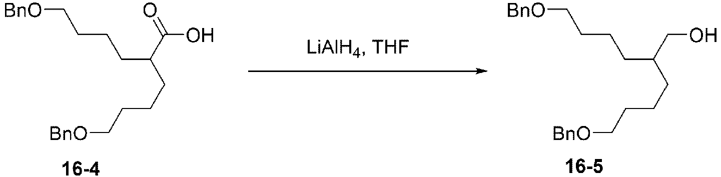

Under ice-water bath conditions, raw material 16-4 (1 g, 2.4 mmol) and anhydrous tetrahydrofuran (30 mL) were added in a 250 mL three-necked flask, and then lithium aluminum tetrahydrogen solid (102 mg, 2.64 mmol) was carefully added in batches. After the addition was completed, the temperature of the reaction solution was slowly raised to room temperature. After 2 hours of reaction, TLC monitoring showed that the raw material was completely reacted. Ethyl acetate (20 mL) was added in the reaction solution for dilution, and then water (2 mL), 15% NaOH aqueous solution (2 mL) and water (5 mL) were slowly added in sequence for quenching. The reaction solution was filtered by suction. The filter cake was washed with ethyl acetate (30 mL) 3 times. The organic phases were combined and spin-dried. Then, silica gel was added for mixing. The product 16-5 was obtained by column chromatography.

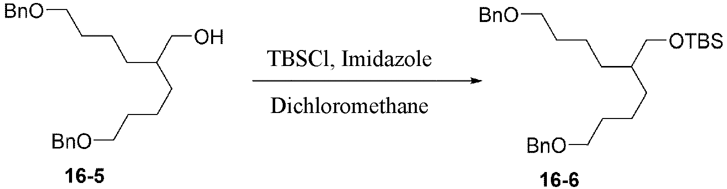

Raw material 16-5 (1.0 g, 2.7 mmol) and imidazole (367 mg, 5.4 mmol) were added in a 50 mL single-necked flask at room temperature, and dissolved completely in dichloromethane (20 mL). Then, t-butyldimethylchlorosilane (611 mg, 4.0 mmol) was added to the reaction system in batches. After the addition was complete, the reaction was allowed to proceed overnight. The reaction was monitored by TLC until the reaction was complete. Water (20 mL) was added into the reaction system for the extraction and liquid separation. The aqueous phase was extracted once with dichloromethane (40 mL). The organic phase was combined, washed once with saturated sodium bicarbonate solution (20 mL) and washed once with saturated sodium chloride solution (20 mL). The organic phase was dried and spin-dried. Then, silica gel was added for mixing. The final product 16-6 was obtained by column chromatography.

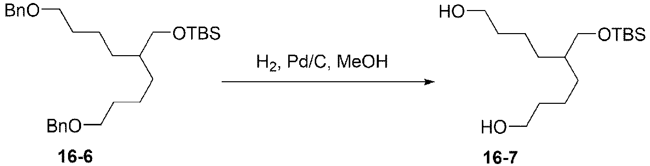

Raw material 16-6 (1.0 g, 1.8 mmol), methanol (20 mL), and palladium carbon containing water (5% palladium content, 100 mg, 10wt%) were added in a 50 mL single-necked flask at room temperature. The atmosphere in the flask was replaced with nitrogen 3 times. Then, a hydrogen-filled balloon was attached. After being replaced with hydrogen for 3 times, the reaction was allowed to proceed overnight. The reaction was monitored by TLC until the raw material was completely reacted. Then, diatomaceous earth was used for suction filtration to remove palladium carbon. The filtrate was spin-dried to obtain a crude product. The crude product was directly used in subsequent reactions without further purification.

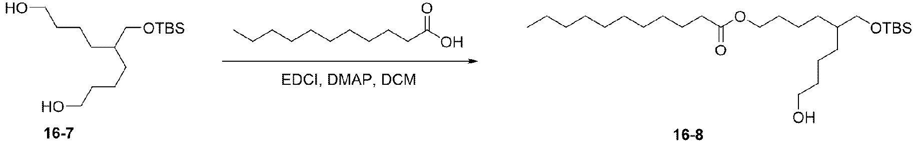

Raw material 16-7 (1.0 g, 3.3 mmol), undecanoic acid (614 mg, 3.3 mmol), 4-dimethylaminopyridine (476 mg, 3.9 mmol) and dichloromethane (40 mL) were added in a 100 mL single-necked flask at room temperature. After mixing and completely dissolving, 1-(3-dimethylaminopropyl)-3-ethylcarbodiimide hydrochloride (757 mg, 3.9 mmol) was added in the reaction system in batches for reaction overnight. The reaction was monitored by TLC until the raw material was completely reacted. Then, ethyl acetate (100 mL) and water (100 mL) were added for the extraction and liquid separation. The organic phase was washed once with saturated sodium chloride (50 mL), dried and spin-dried. Then, silica gel was added for mixing. The product 16-8 was obtained by column chromatography.

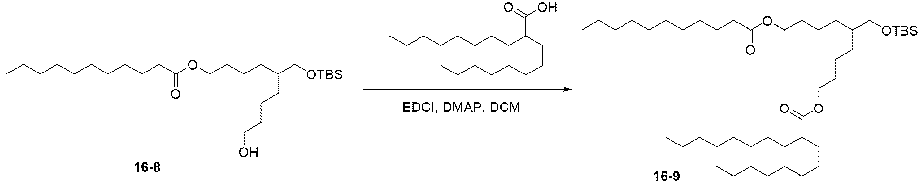

Raw material 16-8 (1.0 g, 2.1 mmol), 2-octyl decanoic acid (722 mg, 2.5 mmol), 1-(3-dimethylaminopropyl)-3-ethylcarbodiimide hydrochloride (480 mg, 2.5 mmol), 4-dimethylaminopyridine (305 mg, 2.5 mmol) and dichloromethane (50 mL) were added in a 100 mL single-necked flask at room temperature to react. After the reaction was allowed to proceed overnight, the reaction was monitored by TLC until the raw material was completely reacted. Then, ethyl acetate (100 mL) and water (100 mL) were added for the extraction and liquid separation. The organic phase was washed once with saturated sodium chloride (50 mL), dried, and spin-dried. Then, silica gel was added for mixing. The product 16-9 was obtained by column chromatography.

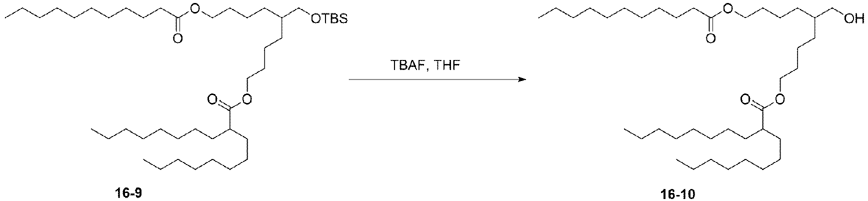

Raw material 16-9 (1.0 g, 1.4 mmol) and tetrahydrofuran (20 mL) were added in a 50 mL single-necked flask at room temperature. After mixing evenly, tetrabutylammonium fluoride in tetrahydrofuran solution (1 mol/L, 2.1 mL) was added dropwise thereto. After the dropwise addition was complete, the reaction was continued at room temperature for 2 hours. The reaction was monitored by TLC until the raw material was completely reacted. Water (50 mL) was slowly added in the reaction system. Most of the organic solvent was removed under reduced pressure, and ethyl acetate (50 mL) was added into the reaction system for the extraction and liquid separation. The organic phase was washed once with a saturated sodium chloride solution (20 mL), dried, and spin-dried. Then, silica gel was added for mixing. The product 16-10 was obtained by column chromatography.

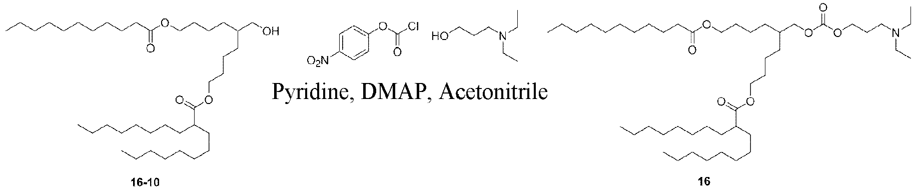

Raw material 16-10 (1.0 g, 1.6 mmol), pyridine (253 mg, 3.2 mmol), 4-dimethylaminopyridine (39 mg, 0.32 mmol), and acetonitrile (20 mL) were added in a 100 mL single-necked flask at room temperature. Then, p-nitrophenyl chloroformate (482 mg, 2.4 mmol) was slowly added thereto. After reacting for 2 hours, the reaction was monitored by TLC until a new fluorescent spot was generated. Then, 3-dimethylamino-1-propanol (628 mg, 4.8 mmol) was added in the reaction system, and the reaction was allowed to proceed overnight. LCMS monitoring showed that the reaction was completed. Then, ethyl acetate (50 mL) and water (50 mL) were added in the reaction system for the extraction and liquid separation. The organic phase was washed once with saturated sodium chloride (30 mL), dried, and spin-dried. Silica gel was added for mixing. The final product Compound 16 was obtained by column chromatography.

The nuclear magnetic data of Compound 16 is as follows: ¹H NMR (400 Mz, CDCl₃) δ 4.20 (t, *J*=6.5 Hz, 2H),4.06(m,6H), 2.59(q, *J*=6.7Hz, 6H), 2.38-2.24 (m,3H), 1.88 (p, *J =* 6.7Hz,2H), 1.62(m,9H), 1.49-1.35 (m,10H), 1.34-1.21(m,38H), 1.06(t, *J*=7.1Hz,6H), 0.94-0.82(m,9H). C₄₇H₉₁NO₇ Exact Mass:781.68, found [M+H]⁺: 782.72.
13.

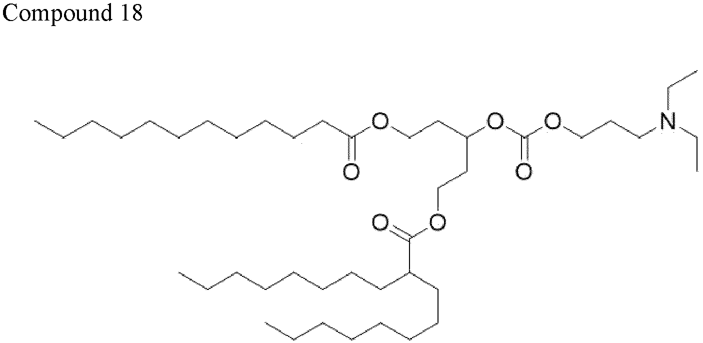

The preparation method of Compound 18 is as follows:

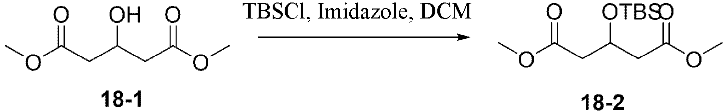

Raw material 18-1 (5.0 g, 28 mmol), imidazole (3.8 g, 56 mmol) and dichloromethane (200 mL) were added in a 500 mL single-necked flask at room temperature, and then t-butyldimethylchlorosilane (6.3 g, 42 mmol) was added in the reaction system in batches; After the addition was complete, the reaction was allowed to proceed overnight. The reaction was monitored by TLC until the reaction was complete. Then, water (200 mL) was added in the reaction system for the extraction and liquid separation. The aqueous phase was extracted once with dichloromethane (50 mL). The organic phase was combined, washed once with a saturated sodium bicarbonate solution (50 mL) and washed once with a saturated sodium chloride solution (100 mL). The organic phase was then dried and spin-dried. Then, silica gel was added for mixing. The product 18-2 was obtained by column chromatography (mobile phase + gradient).

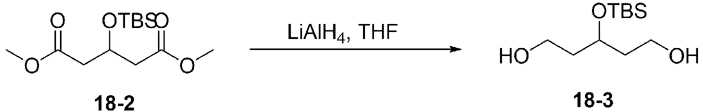

Under ice-water bath conditions, raw material 18-2 (2 g, 6.8 mmol) and anhydrous tetrahydrofuran (50 mL) were added in a 250 mL three-necked flask, and then lithium aluminum tetrahydrogen solid (568 mg, 15.0 mmol) was carefully added in batches. After the addition was completed, the temperature of the reaction solution was slowly raised to room temperature. After reacting for 2 hours, the reaction was monitored by TLC until the raw material was completely reacted. Ethyl acetate (40 mL) was added in the reaction system for dilution, and then water (4 mL), 15% NaOH aqueous solution (4 mL) and water (10 mL) were slowly added for quenching. The reaction solution was filtered by suction. The filter cake was washed three times with ethyl acetate (10 mL). The organic phase was combined and spin-dried. Then, silica gel was added for mixing. The product 18-3 was obtained by column chromatography (mobile phase + gradient).

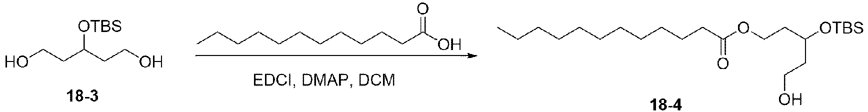

Raw material 18-3 (1.0 g, 4.3 mmol), dodecanoic acid (855 mg, 4.3 mmol), 4-dimethylaminopyridine (634 mg, 5.2 mmol) and dichloromethane (20 mL) were added in a 100 mL single-necked flask at room temperature. After mixing evenly, 1-(3-dimethylaminopropyl)-3-ethylcarbodiimide hydrochloride (991 mg, 5.2 mmol) was added in batches. After reacting overnight, the reaction was monitored by TLC until the raw material was completely reacted. Then, ethyl acetate (50 mL) and water (50 mL) were added in the reaction system for the extraction and liquid separation. The organic phase was washed once with saturated sodium chloride (20 mL), dried and spin-dried. Then, silica gel was added for mixing. The final product 18-4 was obtained by column chromatography (mobile phase + gradient).

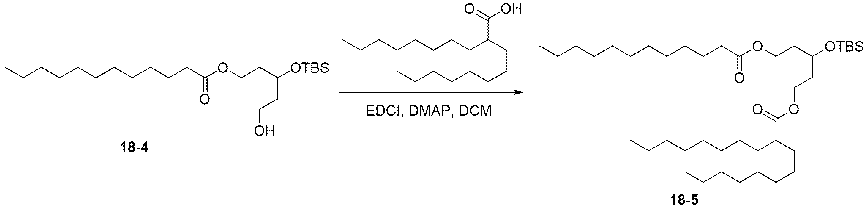

Raw material 18-4 (1.0 g, 2.4 mmol), 2-octyl decanoic acid (682 mg, 2.4 mmol), 1-(3-dimethylaminopropyl)-3-ethylcarbodiimide hydrochloride (538 mg, 2.8 mmol), 4-dimethylaminopyridine (342 mg, 2.8 mmol) and dichloromethane (20 mL) were added in a 100 mL single-necked flask at room temperature. After the reaction was allowed to proceed overnight, the reaction was monitored by TLC until the raw material was completely reacted. Then, ethyl acetate (50 mL) and water (50 mL) were added in the reaction system for the extraction and liquid separation. The organic phase was washed once with saturated sodium chloride (20 mL), dried, and spin-dried. Then, silica gel was added for mixing. The product 18-5 was obtained by column chromatography (mobile phase + gradient).

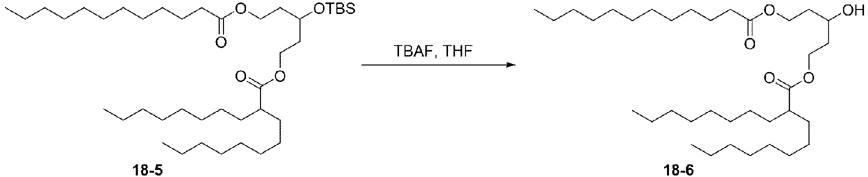

Raw material 18-5 (1.0 g, 1.46 mmol) and tetrahydrofuran (20 mL) were added in a 100 mL single-necked flask at room temperature. After mixing evenly, tetrabutylammonium fluoride in tetrahydrofuran solution (1 mol/L, 2.2 mL) was added dropwise thereto. After the dropwise addition was complete, the reaction was continued at room temperature for 2 hours. The reaction was monitored by TLC until the raw material was completely reacted. Then, water (50 mL) was slowly added in the reaction system to remove most of the organic phase under reduced pressure. Ethyl acetate (50 mL) was added for the extraction and liquid separation. The organic phase was washed once with a saturated sodium chloride solution (20 mL), dried and spin-dried. Then, silica gel was added for mixing. The product 18-6 was obtained by column chromatography (mobile phase + gradient).

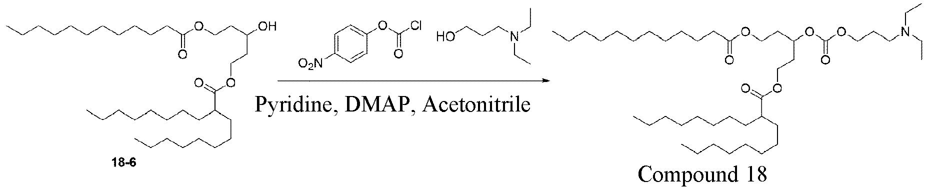

Raw material 18-6 (1.0 g, 1.8 mmol), pyridine (277 mg, 3.5 mmol), 4-dimethylaminopyridine (43 mg, 0.35 mmol), and acetonitrile (20 mL) were added in a 100 mL single-necked flask at room temperature. After dissolving fully, p-nitrophenyl chloroformate (543 mg, 2.7 mmol) was slowly added to react. After 2 hours of reaction, the reaction was monitored by TLC until a new fluorescent spot was generated. Then, 3-dimethylamino-1-propanol (707 mg, 5.4 mmol) was added thereto for reaction overnight. LCMS monitoring showed that the reaction was completed. Then, ethyl acetate (50 mL) and water (50 mL) were added in the reaction system for the extraction and liquid separation. The organic phase was washed 2-3 times with saturated sodium chloride, dried, and spin-dried. Then, silica gel was added for mixing. The final product Compound 18 was obtained by column chromatography (mobile phase + gradient).
14.

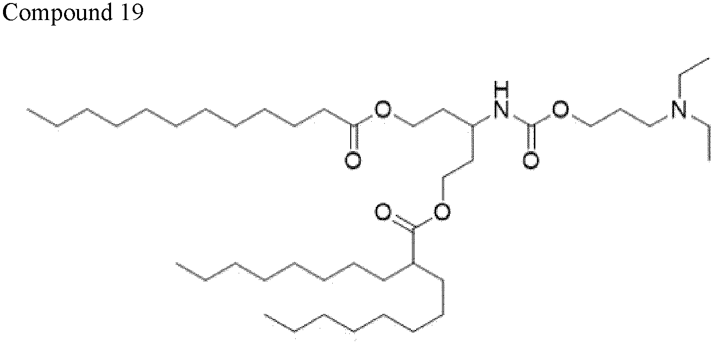

The preparation method of Compound 19 is as follows:

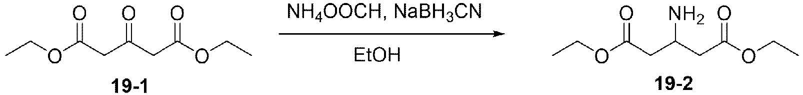

Raw material 19-1 (10 g, 49.4 mmol), ammonium formate (25 g, 396.8 mmol), sodium borohydride (3.45 g, 54.4 mmol), and EtOH (250 mL) were added in a 1000 mL flask in which a thermometer was inserted under ice-water bath conditions. After mixing evenly, the temperature of the reaction solution was slowly raised to room temperature. After reacting for 15 hours, the reaction was monitored by TLC until the raw material was completely reacted. Then, water (100 mL) was carefully added in the reaction system to quench the reaction, and the organic solvent was removed under reduced pressure. The pH value of the system was adjusted to approximately 3 using 3 mol/L hydrochloric acid. The aqueous phase was extracted twice with ethyl acetate (100 mL). The pH of the aqueous phase was carefully adjusted to approximately 10 with potassium carbonate solid. Then, ethyl acetate (100 mL) was used to extract twice. The organic phase was combined, dried, and spin-dried. Then, silica gel was added for mixing. The product 19-2 was obtained by column chromatography (mobile phase + gradient).

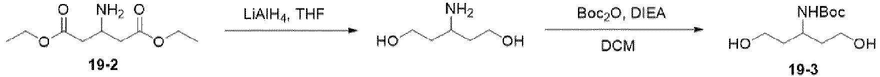

### Step 1:

Raw material 19-2 (2 g, 9.9 mmol) and anhydrous tetrahydrofuran (50 mL) were added in a 250 mL three-necked flask under ice-water bath conditions, and then lithium aluminum tetrahydrogen solid (828 mg, 21.8 mmol) was carefully added thereto in batches. After the addition was completed, the temperature of the reaction solution was slowly raised to room temperature. After 2 hours of reaction, the reaction was monitored by TLC until the raw material was completely reacted. Then, ethyl acetate (20 mL) was added in the reaction system for dilution, and then water (2 mL), 15% NaOH aqueous solution (2 mL) and water (5 mL) were slowly added in sequence for quenching. Then, suction filtration was performed. The filter cake was washed 3 times with ethyl acetate (50 mL). The organic phases were then combined, dried, and spin-dried to obtain a crude product. The obtained crude product was directly used in the next step.

### Step 2:

The crude product obtained in the previous step was dissolved in dichloromethane (50 mL) in a 100 mL single-necked flask under room temperature conditions, and N,N-diisopropylethylamine (2.5 g, 19.8 mmol) and Boc anhydride (3.2 g, 14.9 mmol) were added thereto. The reaction was monitored by TLC until the raw material was completely reacted. Then, water (100 mL) was added in the system for the extraction and liquid separation. The organic phase was washed twice with saturated sodium chloride (30 mL), dried, and spin-dried. Then, silica gel was added for mixing. The product 19-3 was obtained by column chromatography (mobile phase + gradient).

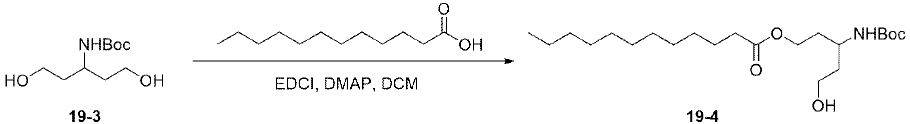

Raw material 19-3 (1.0 g, 4.5 mmol), dodecanoic acid (900 mg, 4.5 mmol), 4-dimethylaminopyridine (671 mg, 5.5 mmol) and dichloromethane (20 mL) were added in a 100 mL single-necked flask at room temperature. After mixing evenly, 1-(3-dimethylaminopropyl)-3-ethylcarbodiimide hydrochloride (1.05 g, 5.5 mmol) was added thereto in batches. The reaction was allowed to proceed overnight. The reaction was monitored by TLC until the raw material was completely reacted. Then, ethyl acetate (50 mL) and water (50 mL) were added in the system for the extraction and liquid separation. The organic phase was washed once with saturated sodium chloride (20 mL), dried, and spin-dried. Then, silica gel was added for mixing. The final product 19-4 was obtained by column chromatography (mobile phase + gradient).

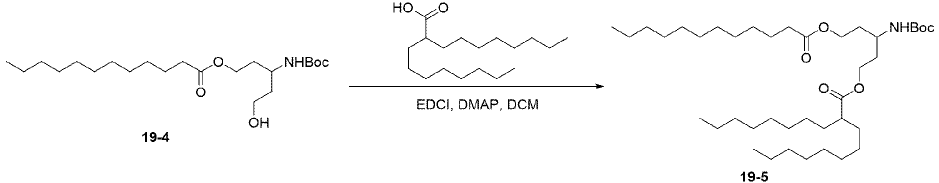

Raw material 19-4 (1.0 g, 2.5 mmol), 2-octyl decanoic acid (710 mg, 2.5 mmol), 1-(3-dimethylaminopropyl)-3-ethylcarbodiimide hydrochloride (574 mg, 3.0 mmol), 4-dimethylaminopyridine (366 mg, 3.0 mmol) and dichloromethane (20 mL) were added in a 100 mL single-necked flask at room temperature. After reacting overnight, the reaction was monitored by TLC until the raw material was completely reacted. Then, ethyl acetate (50 mL) and water (50 mL) were added in the system for the extraction and liquid separation. The organic phase was washed once with saturated sodium chloride (20 mL), dried and spin-dried. Then, silica gel was added for mixing. The product 19-5 was obtained by column chromatography (mobile phase + gradient).

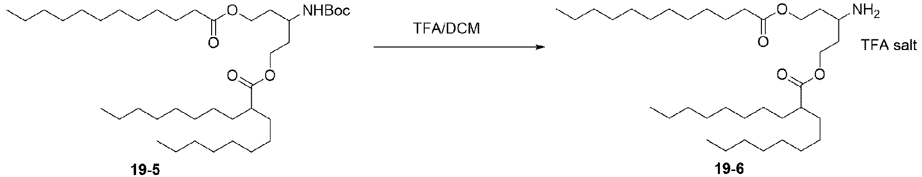

Raw material 19-5 (500 mg, 0.75 mmol), dichloromethane (10 mL) and trifluoroacetic acid (2 mL) were added in a 50 mL flask at room temperature. After mixing and dissolving, the reaction was carried out at room temperature for 2 hours. The reaction was monitored by TLC until the raw material was completely reacted. After the reaction was completed, the system was depressurized to remove the organic solvent to obtain a crude product. The obtained crude product was directly used in the next step without further purification.

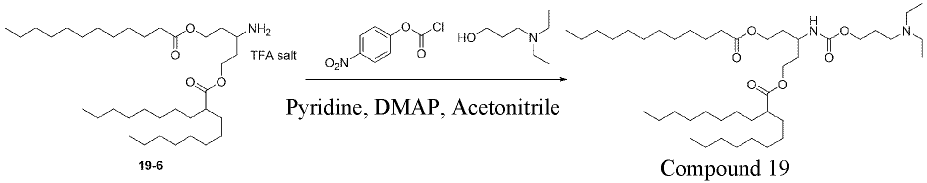

Raw material 19-6 (500 mg, 0.77 mmol), pyridine (182 mg, 2.3 mmol), and 4-dimethylaminopyridine (19 mg, 0.15 mmol) were added in a 100 mL single-necked flask at room temperature and dissolved in acetonitrile (10 mL). Then, p-nitrophenyl chloroformate (232 mg, 1.2 mmol) was slowly added thereto. After reacting for 2 hours, the reaction was monitored by TLC until a new fluorescent spot was generated. Then, 3-dimethylamino-1-propanol (302 mg, 2.31 mmol) was added in the system for reaction overnight. LCMS monitoring showed that the reaction was complete. Then, ethyl acetate (30 mL) and water (30 mL) were added in the system for the extraction and liquid separation. The organic phase was washed 2-3 times with saturated sodium chloride, dried, and spin-dried. Then, silica gel was added for mixing. The final product Compound 19 was obtained by column chromatography (mobile phase + gradient).
15

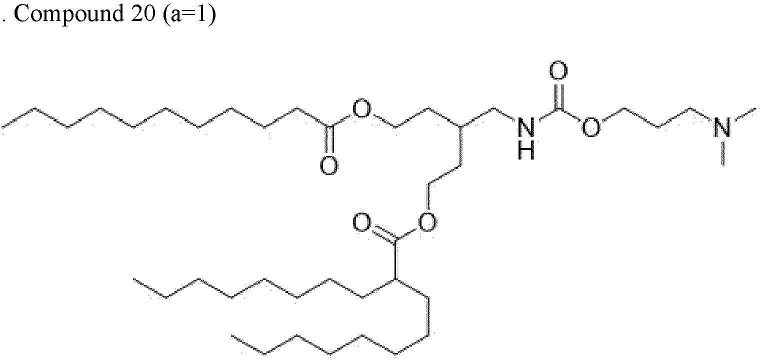

The preparation method of Compound 20 is as follows:

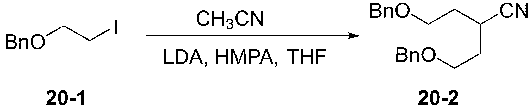

Under nitrogen protection, acetonitrile (1.6 mL, 31.6 mmol), hexamethylphosphoric triamide (11 mL), and anhydrous tetrahydrofuran (23 mL) were added in a 100 mL three-necked flask respectively. After mixing evenly, the temperature was lowered to -78 °C. Under low temperature protection, LDA solution (15.2 mL, 2.0 mol/L, tetrahydrofuran/n-hexane) was slowly added dropwise into the three-necked flask. The reaction solution was stirred for 30 minutes after the dropwise addition was completed. Then, 20-1 (6.86 g, 26.2 mmol) in tetrahydrofuran (25 mL) solution was added dropwise in the system, and the reaction was continued at low temperature for 2 hours. Then, a second batch of LDA solution (15.2 mL, 2.0 mol/L, tetrahydrofuran/n-hexane) was added dropwise in the system. The reaction was stirred for 30 minutes after the dropwise addition was completed. Then, a second batch of 20-1 (6.86 g, 26.2 mmol) in tetrahydrofuran (25 mL) solution was added dropwise in the system, and the reaction was continued at low temperature for 2 hours. The reaction was monitored by TLC until the raw material was completely reacted. At low temperature, saturated ammonium chloride solution (5 mL) solution was used to quench the reaction. The temperature of the system was raised to room temperature. The organic phase was evaporated by rotary evaporation under reduced pressure. 50 mL of water was added to dilute the system, and the system was extracted with ethyl acetate (100 mL). The organic phase was washed once with saturated sodium chloride solution (50 mL), dried, and spin-dried. Then, silica gel was added for mixing. The product 20-2 was obtained by column chromatography (mobile phase + gradient).

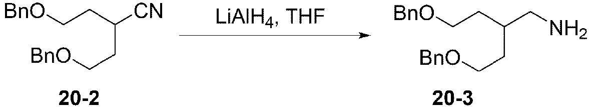

In an ice water bath, under nitrogen protection, lithium aluminum tetrahydrogen (737 mg, 19.4 mmol) and anhydrous tetrahydrofuran (50 mL) were added in a 250 mL flask in which a thermometer inserted and dissolved. After mixing evenly, raw material 20-2 (2 g, 6.5 mmol) in tetrahydrofuran (20 mL) solution was added dropwise in the system. The temperature of the reaction solution was slowly raised to room temperature, and the reaction was carried out at 70 ° C for 16 hours. The reaction was monitored by TLC until the raw material was completely reacted. Then, the temperature was lowered to 0 °C. Water (1 mL), 10% sodium hydroxide solution (2 mL) and water (1 mL) were carefully added to quench the reaction. The mixture was filtered by suction, and the filtered viscous solid was washed 3 times with ethyl acetate (50 mL). The organic phase was combined, dried, and spin-dried to obtain a crude product. The crude product was used directly in the next step without further purification.

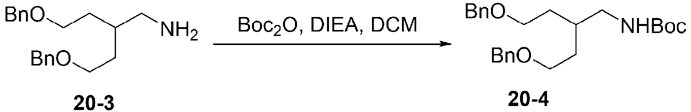

The crude product obtained in the previous step was dissolved in dichloromethane (50 mL) in a 100 mL single-necked flask at room temperature, and then N, N-diisopropylethylamine (1.7 g, 13 mmol) and Boc anhydride (2.1 g, 9.8 mmol) were added thereto for reaction. The reaction was monitored by TLC until the raw material was completely reacted. Then, water (100 mL) was added thereto for the extraction and liquid separation. The organic phase was washed twice with saturated sodium chloride (40 mL), dried, and spin-dried. Then, silica gel was added for mixing. The product 20-4 was obtained by column chromatography (mobile phase + gradient).

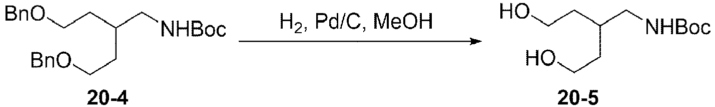

Raw material 20-4 (1.0 g, 2.4 mmol), methanol (20 mL), and palladium carbon containing water (5% palladium content, 100 mg, 10 wt%) were added in a 50 mL single-necked flask at room temperature. The atmosphere in the flask was replaced with nitrogen three times. Then, a hydrogen-filled balloon was attached. After being replaced with hydrogen for 3 times, the reaction was allowed to proceed overnight. The reaction was monitored by TLC until the raw material was completely reacted. Then, diatomaceous earth was used for suction filtration to remove palladium carbon. The filtrate was spin-dried to obtain a crude product. The crude product was used directly in subsequent reactions without further purification.

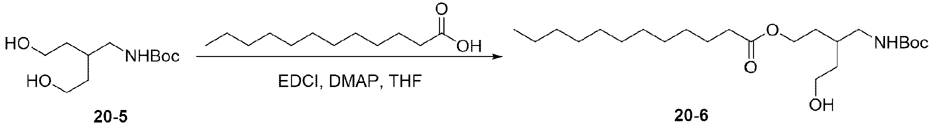

Raw material 20-5 (1.0 g, 4.3 mmol), dodecanoic acid (860 mg, 4.3 mmol), 4-dimethylaminopyridine (634 mg, 5.2 mmol) and dichloromethane (20 mL) were added in a 100 mL single-necked flask at room temperature. After mixing evenly, 1-(3-dimethylaminopropyl)-3-ethylcarbodiimide hydrochloride (991 mg, 5.2 mmol) was added in batches to react. After reacting overnight, the reaction was monitored by TLC until the raw material was completely reacted. Then, ethyl acetate (50 mL) and water (50 mL) were added in the system for the extraction and liquid separation. The organic phase was washed twice with a saturated sodium chloride solution (20 mL), dried, and spin-dried. Then, silica gel was added for mixing. The product 20-6 was obtained by column chromatography (mobile phase + gradient).

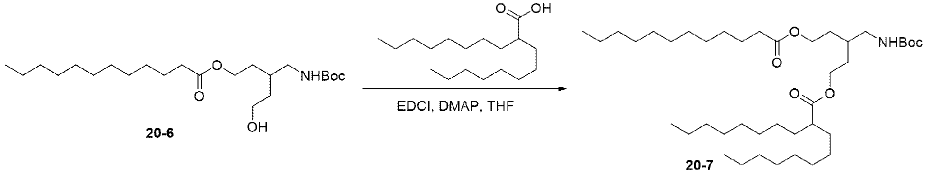

Raw material 20-6 (1.0 g, 2.4 mmol), 2-octyldecanoic acid (682 mg, 2.4 mmol), 1-(3-dimethylaminopropyl)-3-ethylcarbodiimide hydrochloride (555 mg, 2.9 mmol), 4-dimethylaminopyridine (354 mg, 2.9 mmol) and dichloromethane (20 mL) were added in a 100 mL single-necked flask at room temperature, and the reaction was carried out overnight. The reaction was monitored by TLC until the raw material was completely reacted. Then, ethyl acetate (50 mL) and water (50 mL) were added in the system for the extraction and liquid separation. The organic phase was washed twice with saturated sodium chloride (20 mL), dried, and spin-dried. Then, silica gel was added for mixing. The product 20-7 was obtained by column chromatography (mobile phase + gradient).

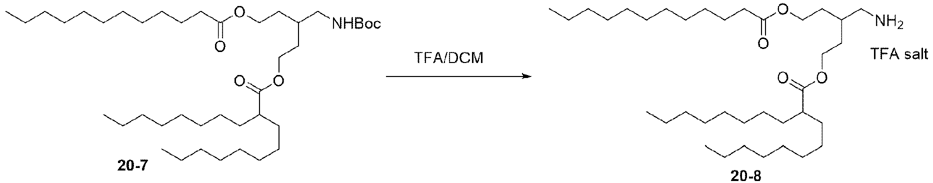

Raw material 20-7 (500 mg, 0.73 mmol), dichloromethane (10 mL) and trifluoroacetic acid (2 mL) were added in a 50 mL flask at room temperature. After mixing evenly, the reaction was carried out at room temperature for 2 hours. The reaction was monitored by TLC until the raw material was completely reacted. Then, the system was depressurized to remove the organic solvent to obtain a crude product. The obtained crude product was directly used in the next step without further purification.

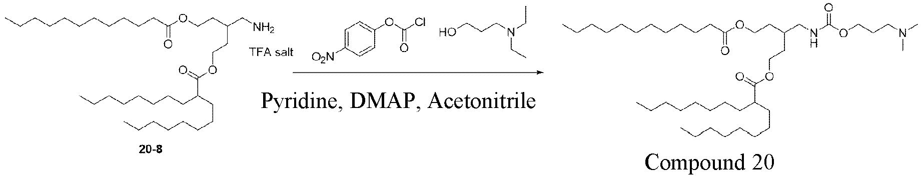

Raw material 20-8 (1.0 g, 1.5 mmol), pyridine (356 mg, 4.5 mmol), 4-dimethylaminopyridine (37 mg, 0.3 mmol), and acetonitrile (20 mL) were added in a 100 mL single-necked flask at room temperature. After dissolving fully, p-nitrophenyl chloroformate (462 mg, 2.3 mmol) was slowly added thereto. After reacting for 2 hours, the reaction was monitored by TLC until a new fluorescent spot was generated. Then, 3-dimethylamino-1-propanol (590 mg, 4.5 mmol) was added in the system for reaction overnight. LCMS monitoring showed that the reaction was completed. Then, ethyl acetate (60 mL) and water (60 mL) were added in the system for the extraction and liquid separation. The organic phase was washed once with saturated sodium chloride (30 mL), dried, and spin-dried. Then, silica gel was used for mixing. The final product Compound 20 was obtained by column chromatography (mobile phase + gradient).
16.

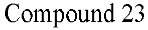

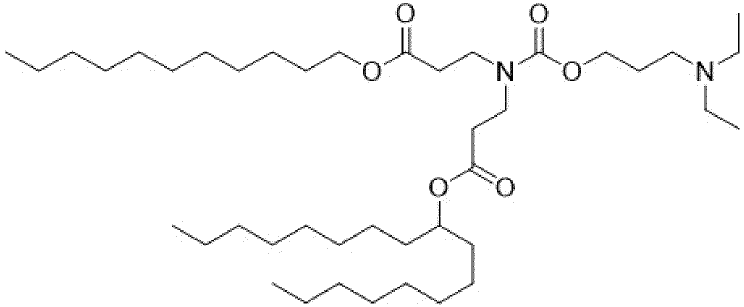

The preparation scheme of Compound 23 is as follows:

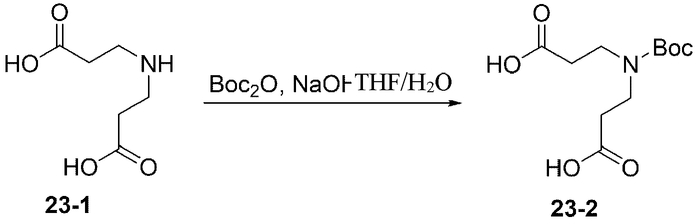

Raw material 23-1 (10.0 g, 62.08 mmol) and sodium hydroxide aqueous solution (300 mL, 1 mol/L) were added in a 1000 mL single-necked flask at room temperature. After mixing evenly, Boc anhydride (22.1 g, 101 mmol) in tetrahydrofuran (200 mL) solution was slowly added dropwise thereto within 1 hour. After the dropwise addition was completed, the reaction was continued at room temperature for 2 hours, The reaction was monitored by TLC until the raw material was completely reacted. 0.2 mol/L phosphoric acid solution was carefully added in the reaction mixture until the pH was approximately equal to 4. The organic solvent was rotary evaporated under reduced pressure, and then extracted once with dichloromethane (300 mL). The organic phase was washed with saturated sodium chloride aqueous solution (200 mL), dried, and then spin-dried. Then, silica gel was added for mixing. The product 23-2 was obtained by column chromatography (mobile phase + gradient).

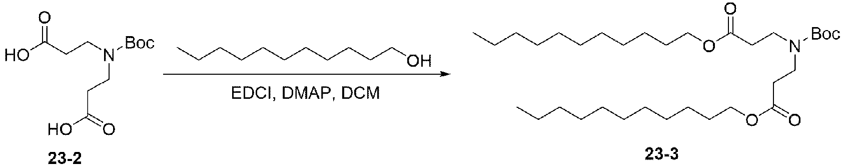

Raw material 23-2 (5.0 g, 19. mmol), undecanol (8.2 g, 47.9 mmol), 1-(3-dimethylaminopropyl)-3-ethylcarbodiimide hydrochloride (9.2 g, 47.9 mmol), 4-dimethylaminopyridine (468 mg, 3.84 mmol) and dichloromethane (100 mL) were added in a 250 mL single-necked flask at room temperature to react. After the reaction was allowed to proceed overnight, the reaction was monitored by TLC until the raw material was completely reacted. Then, acetic acid (5 mL) and methanol (10 mL) were carefully added in the reaction system to quench the reaction, and water (100 mL) was added to dilute the system. Then, the organic solvent was rotary evaporated under reduced pressure, and ethyl acetate (100 mL) was used for the extraction and liquid separation. The organic phase was washed once with a saturated sodium chloride solution (50 mL), dried, and spin-dried. Then, silica gel was added for mixing. The product 23-3 was obtained by column chromatography (mobile phase + gradient).

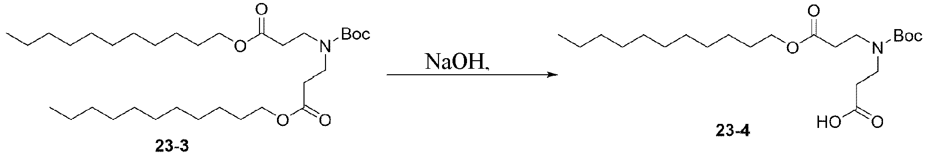

Raw material 23-3 (5 g, 8.8 mmol) and ethanol (100 mL) were added in a 250 mL flask at room temperature. After mixing evenly, an aqueous sodium hydroxide solution (351 mg, 8.8 mmol, dissolved in 30 mL of water) was slowly added dropwise in the system. After the dropwise addition was completed, the reaction was continued at room temperature for 4 hours. The reaction was monitored by TLC. 1 mol/L hydrochloric acid was slowly added dropwise into the system until the pH of the system was approximately equal to 5. The organic phase was removed under reduced pressure. Water (100 mL) was added to dilute the system, and ethyl acetate (100 mL) was used for the extraction and liquid separation. The organic phase was washed once with a saturated sodium chloride solution (50 mL), dried, and spin-dried. Then, silica gel was added for mixing. The product 23-4 was obtained by column chromatography (mobile phase + gradient).

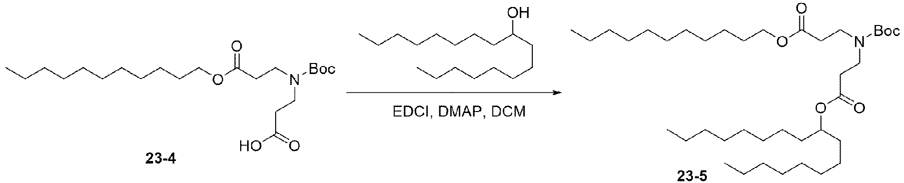

Raw material 23-4 (2.0 g, 4.8 mmol), 9-heptadecanol (1.5 g, 5.8 mmol), 1-(3-dimethylaminopropyl)-3-ethylcarbodiimide hydrochloride (1.4 g, 7.2 mmol), 4-dimethylaminopyridine (878 mg, 7.2 mmol) and dichloromethane (50 mL) were added in a 100 mL single-necked flask at room temperature to react. After the reaction was allowed to proceed overnight, the reaction was monitored by TLC until the raw material was completely reacted. Ethyl acetate (100 mL) and water (100 mL) were then added in the system for the extraction and liquid separation. The organic phase was washed once with saturated sodium chloride (30 mL), dried, and spin-dried. Then, silica gel was added for mixing. The product 23-5 was obtained by column chromatography (mobile phase + gradient).

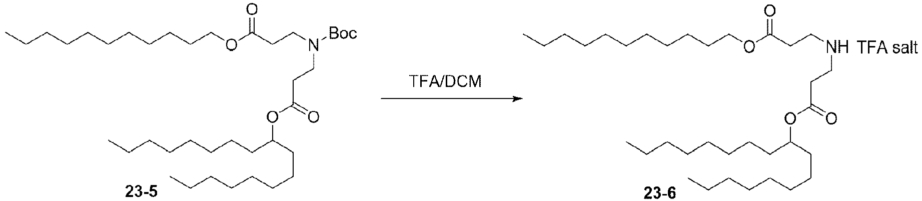

Raw material 23-5 (1.0 g, 1.5 mmol), dichloromethane (20 mL) and trifluoroacetic acid (4 mL) were added in a 50 mL flask at room temperature. After mixing evenly, and the reaction was carried out at room temperature for 2 hours. The reaction was monitored by TLC, until the raw material was completely reacted. Then, the organic solvent was removed under reduced pressure to obtain a crude product. The obtained crude product was directly used in the next step without further purification.

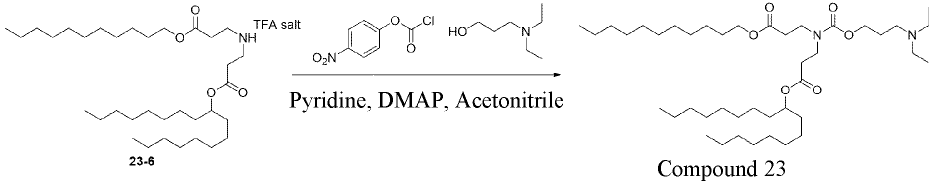

Raw material 23-6 (500 mg, 0.88 mmol), pyridine (209 mg, 2.64 mmol), 4-dimethylaminopyridine (21 mg, 0.18 mmol), and acetonitrile (10 mL) were added in a 100 mL single-necked flask at room temperature to dissolve. Then, p-nitrophenyl chloroformate (265 mg, 1.32 mmol) was slowly added thereto. After reacting for 2 hours, the reaction was monitored by TLC until a new fluorescent spot was generated. Then, 3-dimethylamino-1-propanol (346 mg, 2.64 mmol) was added in the system for reaction overnight. LCMS monitoring showed that the reaction was completed. Then, ethyl acetate (30 mL) and water (30 mL) were added in the system for the extraction and liquid separation. The organic phase was washed once with a saturated sodium chloride solution (20 mL), dried, and spin-dried. Then, silica gel was used for mixing. The final product Compound 23 was obtained by column chromatography (mobile phase + gradient).

Hereinafter, the technical effects achieved by applying the cationic lipid compound of the present invention in the delivery system for mRNA (LNP) will be described based on Compound 1, Compound 2, Compound 3, Compound 4, Compound 5, Compound 6, Compound 7, Compound 9, Compound 12, Compound 13, Compound 15, and Compound 16.

### Example 1

### Preparation and detection of lipid nanoparticle formulation

### 1. Preparation steps

The above-mentioned Compound 1 of the present invention with DSPC, cholesterol and DMG-PEG2000 was dissolved in anhydrous ethanol at a molar ratio of 50:10:38.5:1.5 to prepare an ethanol lipid solution.

Luciferase (Luc) or interleukin 2 (IL2) mRNA were diluted with 50mM citrate buffer (pH=4) to obtain an aqueous mRNA solution.

Lipid nanoparticles were prepared by mixing the ethanol lipid solution and the aqueous mRNA solution in a volume ratio of 1:3 by a microfluidic device, standing for dialysis for 18h. The ethanol was removed, and the liquid replacement of PBS was completed. Finally, the lipid nanoparticle solution was filtered through a 0.22µm sterile filter and concentrated through ultrafiltration, to finally obtain a lipid nanoparticle formulation of Compound 1 encapsulating luciferase or interleukin 2 mRNA therein.

In addition, an MC3 lipid nanoparticle formulation was prepared as a control.

In addition, Compound 2, Compound 3, Compound 4, Compound 5, Compound 6, Compound 7, Compound 9, Compound 12, Compound 13, Compound 15, and Compound 16 were prepared into lipid nanoparticle formulations, and the specific preparation steps thereof were the same as those of Compound 1.

### 2. Detection steps

Particle size, polydispersity index (PDI) and potential (Zeta) of the lipid nanoparticles were measured using Litesizer^{™}500 (Anton Paar, Austria). The particle size and potential were measured in 0.1% PBS. The encapsulation efficiency of the lipid nanoparticle formulation was obtained by RiboGreen detection method. The test results are shown in Table 1:

**Table 1**

| Formulation name | Particle size | PDI (%) | Zeta | EE (%) |
|---|---|---|---|---|
| MC3-Luc | 95.69±2.45 | 16.25±1.91 | 35.55±0.49 | 86.75±6.29 |
| Compound 1-Luc | 90.43±3.07 | 14.95±7.57 | 22.30±2.12 | 95.00±0.85 |

It can be seen from Table 1 that Compound 1 of the present invention as an ionic lipid can form lipid nanoparticle, and its basic physicochemical properties are almost the same as those of the control MC3.

### Example 2

### Cell transfection of lipid nanoparticle formulations

The lipid nanoparticle formulation of Compound 1 encapsulating Luc mRNA in Example 1 was prepared. At the same time, the MC3 lipid nanoparticle formulation was prepared as a control.

The above two formulations were diluted to 320 ng/mL with culture medium, and Hek293 cells were added thereto. After 24 hours of transfection, the culture medium was removed, and then rinsed with PBS. Subsequently, the cell lysis solution was added and incubated for 10 minutes. After the lysis was completed, the luciferase substrate was added, and the luminescent signal was detected by a multifunctional microplate reader with a chemiluminescent module. The test results were shown in Fig. 1.

It can be seen from Fig. 1 that: compared with the control (MC3 lipid nanoparticle formulation), the cell transfection ability of the lipid nanoparticle formulation of Compound 1 of the present invention was enhanced by more than ten times. Accordingly, the compounds of the present invention have excellent delivery efficiency when used in the delivery system for mRNA.

### Example 3

In vivo transfection of the lipid nanoparticle formulation of Compound 1

BALB/c mice were divided into four groups, with 4 mice in each group. Each group was injected with MC3 or lipid nanoparticle formulation of Compound 1 intravenously (see (a) in Fig. 2, 10 µg/mouse) or intramuscularly (see (b) in Fig. 2, 5 µg/mouse) once. Luciferase substrate was injected intraperitoneally 6 hours later. After waiting for 10 minutes, the mice were dissected, and the liver, lung, kidney, and spleen organs were sampled. The signals were observed and quantified using a live imaging device.

The experimental results were shown in Fig. 2. It can be seen from Fig. 2 that: compared with the control (MC3 lipid nanoparticle formulation), the expression of the lipid nanoparticle formulation of Compound 1 of the present invention in the spleen is significantly higher than that of MC3. Accordingly, the delivery organ of the cationic lipid compound of the present invention is the spleen, indicating that it has organ-targeting property.

### Example 4

In vivo transfection of lipid nanoparticle formulations of Compound 1, Compound 2, Compound 3, Compound 4, Compound 5, Compound 6, Compound 7, Compound 9, Compound 12, Compound 13, Compound 15, and Compound 16 prepared in Example 1

BALB/c mice were divided into different groups, with 3 mice in each group. Each group was intramuscularly injected with MC3 lipid nanoparticle formulation, or the lipid nanoparticle formulations prepared in Example 1 at a dose of 2.5 µg/mouse. Luciferase substrate was injected intraperitoneally 6 h later, and the injection site signal was observed and quantified using a live imaging device after waiting for 10 min. Subsequently, the mice were dissected, and the liver, spleen, lymph nodes and other organs were sampled, and the signals of each organ were quantified again.

The experimental results are shown in Fig. 3 and Fig. 4. It can be seen from Fig. 3 that: the lipid nanoparticle formulations prepared by each compound in Example 1 all expressed a certain level of protein in vivo, indicating that their delivery efficiencies are high.

Fig. 4A, Fig.4B, and Fig.4C are statistical diagrams of transfection effects of lipid nanoparticle formulations prepared by the compounds of the present invention and the control at different sites; wherein, FIG. 4A shows the quantitative results of liver distribution in mice after 6 hours of intramuscular injection; FIG. 4B shows the quantitative results of spleen distribution in mice after 6 hours of intramuscular injection; FIG. 4C shows the quantitative results of lymph node distribution in mice after 6 hours of intramuscular injection. It can be seen from Fig. 4A, Fig. 4B, and Fig. 4C that: compared with the control (MC3 lipid nanoparticle formulation), the expression of the lipid nanoparticle formulations of the compounds of the examples of the present invention in the spleen and lymph node sites is significantly higher than that of MC3. Accordingly, the delivery organ of the cationic lipid compound of the present invention is the spleen and/or lymph node, indicating that it has organ-targeting property.

### Example 5

In vivo transfection of hIL2 mRNA with lipid nanoparticle formulation for immune activation

BALB/c mice were divided into 2 groups according to body weight, with 4 mice in each group. Each group was intravenously injected once with MC3 lipid nanoparticle formulation or lipid nanoparticle formulation of Compound 1 at a dose of 10 µg/mouse. Blood was sampled at 3 h, 6 h, and 24 h after administration. The concentration of hIL-2 (see (a) in Fig. 5) and IFN-γ (see (b) in Fig. 5) IN plasma were detected by Elisa.

It can be seen from Fig. 5 that: compared with the control (MC3 lipid nanoparticle formulation), when the mouse was injected with lipid nanoparticle formulation of the Compound 1 of the present invention, although the concentration of hIL-2 in mouse plasma was low, the content of IFN-γ in plasma was significantly higher than that of the control by several times. Therefore, the lipid nanoparticle formulation prepared by the Compound 1 of the present invention exhibited excellent immune activation properties.

In summary, the cationic lipid compound developed by the present invention has the technical effects of high delivery effect, organ-targeted delivery, and excellent immune activation properties when used for mRNA delivery.

The above described are only preferred embodiments of the present invention and are not intended to limit the present invention in any other form. Any simple modifications, equivalent substitutions and improvements made to the above examples based on the technical essence of the present invention shall still fall within the scope of the technical solutions of the present invention.

## Claims

1. A cationic lipid compound, wherein the cationic lipid compound has a structure represented by the following formula (I): 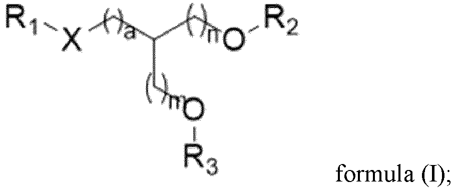 wherein, in the formula (I): X is O or N; n is 2-4; m is 2-4; a is 0 or 1; R₁ is a chain structure comprising a tertiary amine; R₂ is a linear fatty acyl group or a branched chain fatty acyl group; R₃ is a branched chain fatty acyl group.

2. The cationic lipid compound according to claim 1, wherein the chain structure comprising a tertiary amine is a chain structure of a tertiary amine.

3. The cationic lipid compound according to claim 1, wherein the R₁ is selected from any one of the following groups:

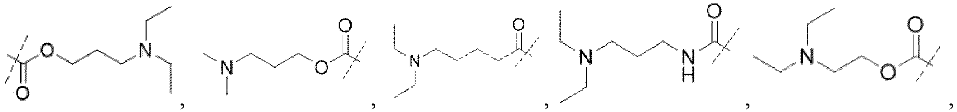

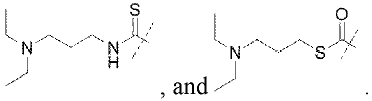

4. The cationic lipid compound according to claim 1, wherein the linear fatty acyl group is a C8-C16 linear fatty acyl group.

5. The cationic lipid compound according to claim 4, wherein the linear fatty acyl group is a C10-C14 linear fatty acyl group.

6. The cationic lipid compound according to claim 1, wherein the linear fatty acyl group is any one of the following groups:

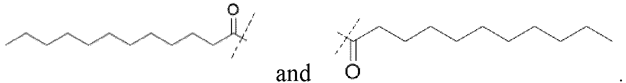

7. The cationic lipid compound according to claim 1, wherein in the branched chain fatty acyl group: the number of the long-chain C atoms is less than or equal to 16, and the number of the short-chain C atoms is less than or equal to the number of the long-chain C atoms.

8. The cationic lipid compound according to claim 7, wherein the branched chain fatty acyl group is any one of the following groups:

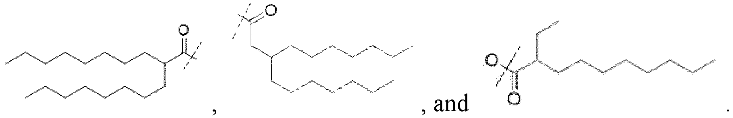

9. The cationic lipid compound according to any one of claims 1 to 7, wherein the cationic lipid compound is any one of the following compounds: 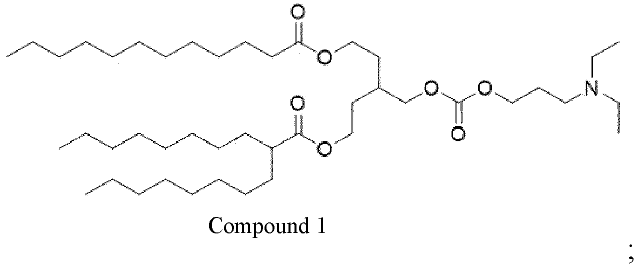 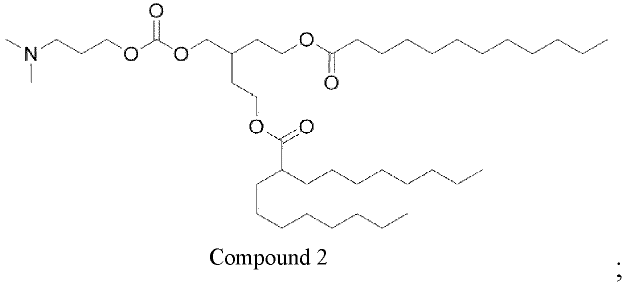 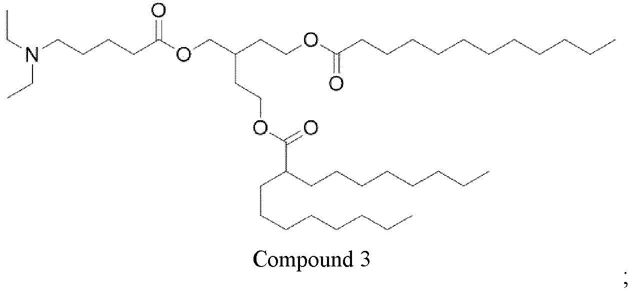 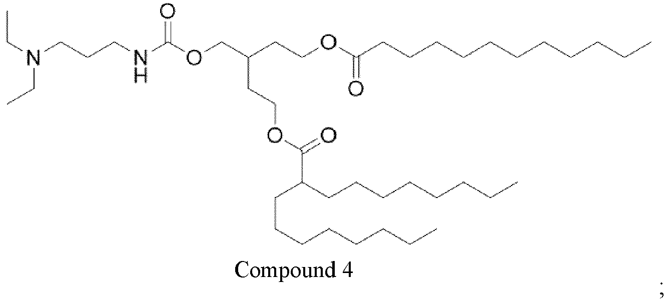 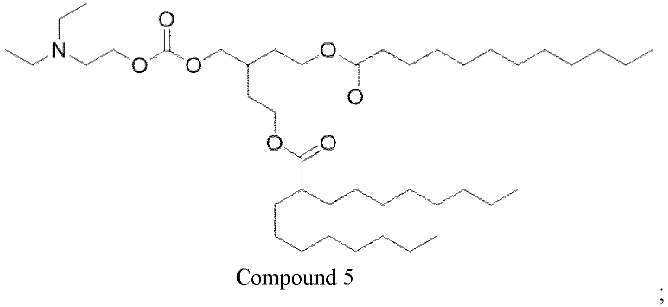 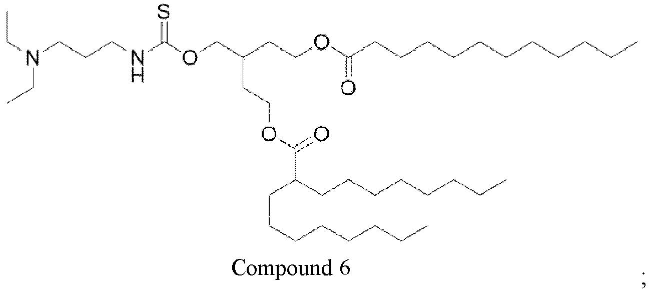 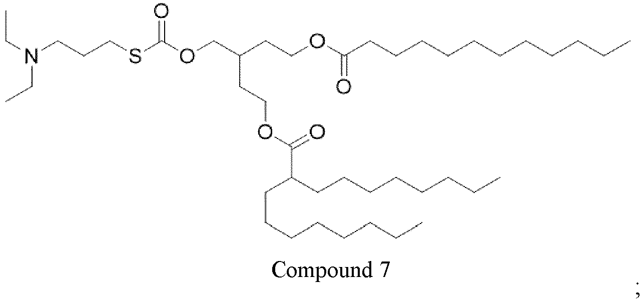 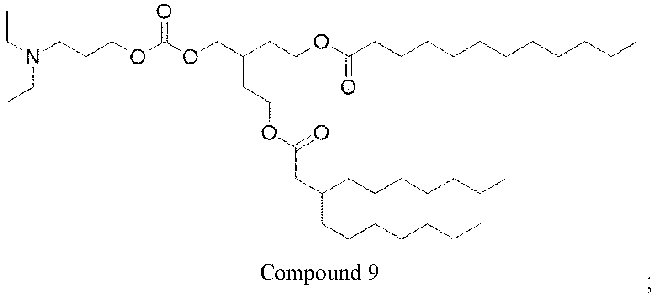 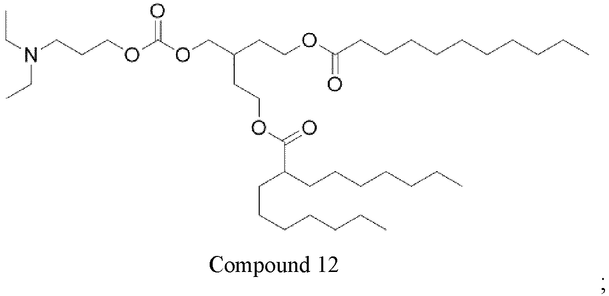 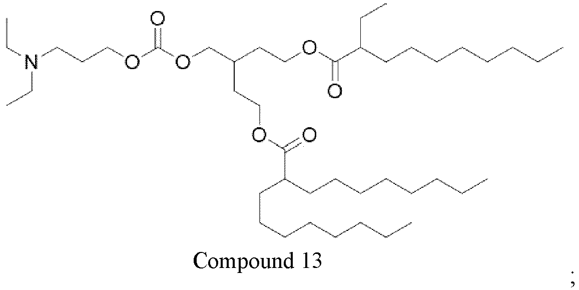 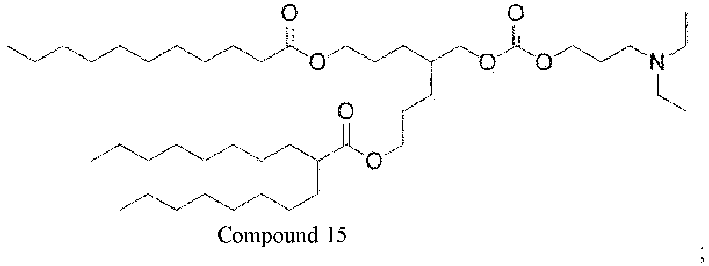 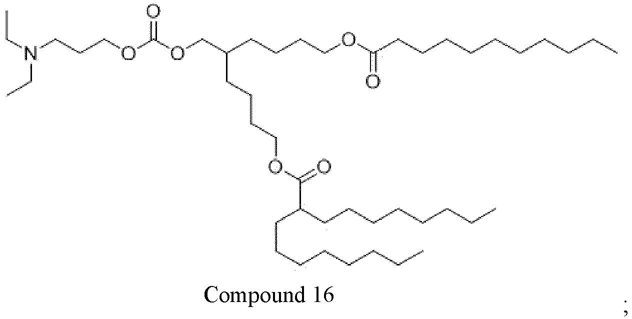 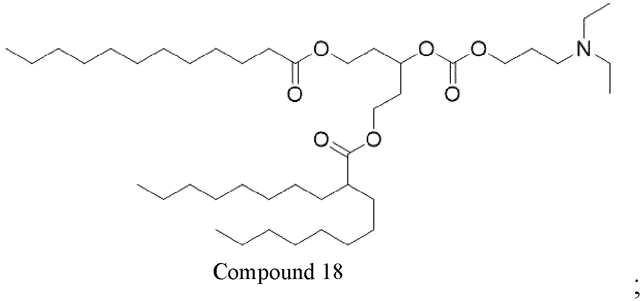 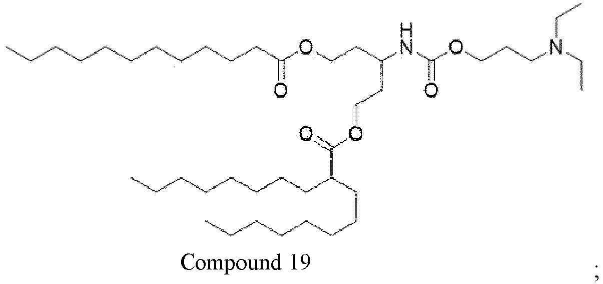 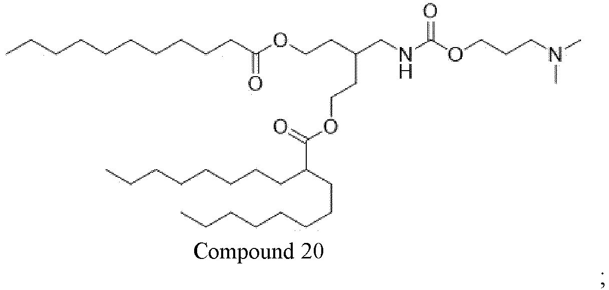 and

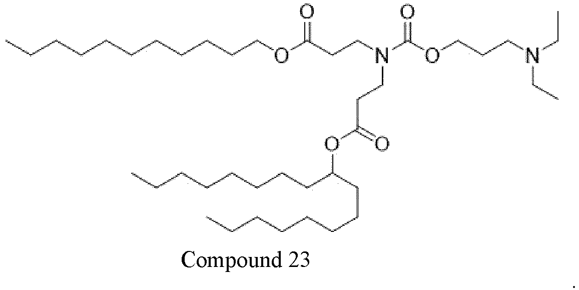

10. A preparation method of the cationic lipid compound according to claim 9, wherein the chemical reaction procedure for preparing the Compounds 1-5 and Compound 7 is as follows: 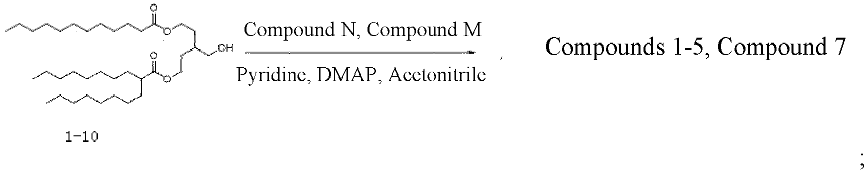
wherein when the prepared compound is Compound 1, the Compound N and the Compound M are selected as: 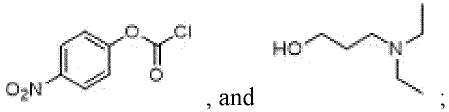
when the prepared compound is Compound 2, the Compound N and the Compound M are selected as: 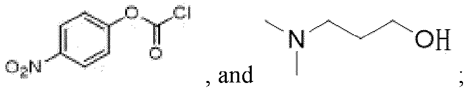
when the prepared compound is Compound 3, the Compound N and the Compound M are selected as: 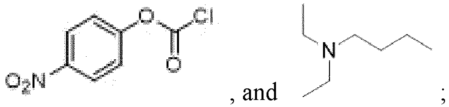
when the prepared compound is Compound 4, the Compound N and the Compound M are selected as: 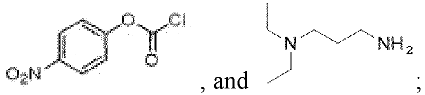
when the prepared compound is Compound 5, the Compound N and the Compound M are selected as: 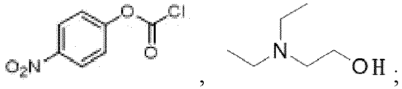
when the prepared compound is Compound 7, the Compound N and the Compound M are selected as: 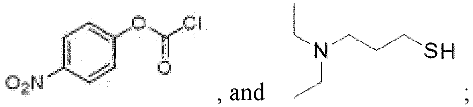
wherein, the preparation steps of the Compound 1 comprise: raw material 1-10, pyridine, 4-dimethylaminopyridine and acetonitrile are added into a reaction vessel under room temperature; and then p-nitrophenyl chloroformate is added thereto for reaction; after the reaction is carried out for a set time, a TLC spot plate shows that a new main spot is formed; and then 3-diethylamino-1-propanol is added into the reaction system for reaction; after the reaction is completed, extraction and liquid separation are carried out; the organic phase is washed, dried and purified, to obtain a colorless oily product of Compound 1.

11. The preparation method of the cationic lipid compound according to claim 10, wherein the chemical formula for preparing the raw material 1-10 is as follows: 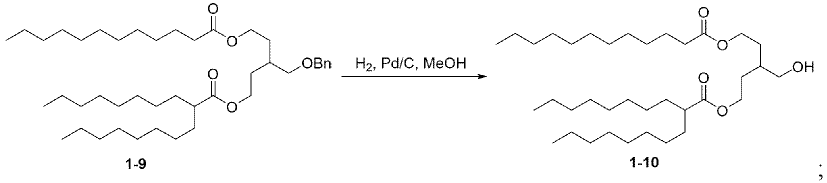 wherein the preparation steps of the raw material 1-10 comprise: raw material 1-9, methanol and palladium-carbon containing water are added into a reaction vessel under room temperature; after the gas in reaction vessel is sequentially replaced with inert gas and hydrogen gas for multiple times, a reaction is carried out; after the reaction is completed, a suction filtration treatment is carried out to remove palladium-carbon; the filtrate is spin-dried to obtain a colorless oily product of raw material 1-10.

12. A preparation method of the cationic lipid compound according to claim 9, wherein the chemical reaction procedure for preparing Compound 9, Compound 12, Compound 13, Compound 15, Compound 16 and Compound 18 is as follows: 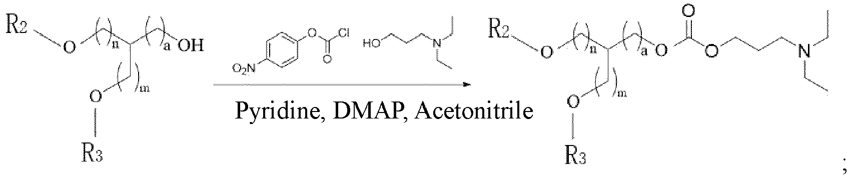 wherein the preparation steps comprise: raw materials 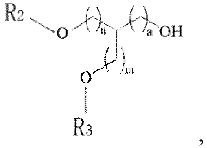 pyridine, 4-dimethylaminopyridine, and acetonitrile are added into a vessel under room temperature; after dissolution, p-nitrophenyl chloroformate is added thereto for reaction; the reaction is monitored by TLC, until a new fluorescent spot is generated; and then 3-dimethylamino-1-propanol is added into the reaction system for reaction; after the reaction is completed, extraction and liquid separation are carried out, and the organic phase is washed, dried and purified, to obtain the required compound.

13. A preparation method of the cationic lipid compound according to claim 9, wherein the chemical reaction procedure for preparing the Compound 19 or Compound 20 is as follows: 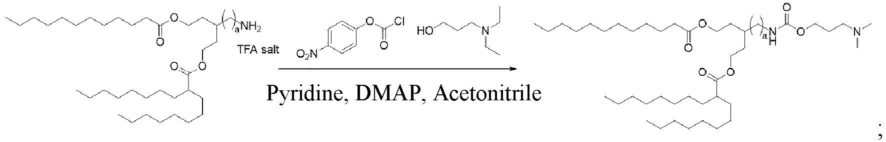 preferably, the preparation steps of the Compound 19 or Compound 20 comprise: raw material 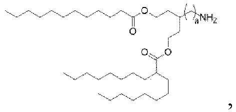 pyridine, 4-dimethylaminopyridine, and acetonitrile are added into a reaction vessel; after full dissolution, p-nitrophenyl chloroformate is added thereto for reaction; the reaction is monitored by TLC, until a new fluorescent spot is generated; and then 3-dimethylamino-1-propanol is added into the reaction system for reaction overnight; after the reaction is completed, extraction and liquid separation are carried out, and the organic phase is washed, dried and purified, to obtain the Compound 19 or Compound 20.

14. A preparation method of the cationic lipid compound according to claim 9, wherein the chemical formula for preparing the Compound 6 is as follows: 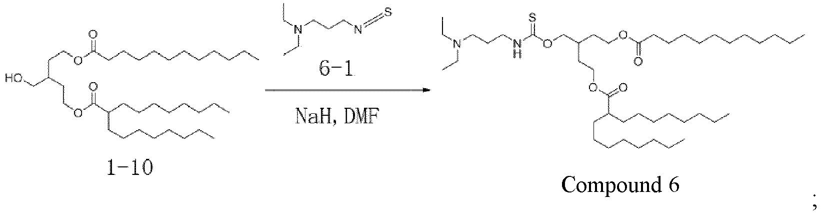 wherein the preparation steps of the Compound 6 are as follows: raw material 1-10 and DMF are added into a reaction vessel, mixed and dissolved, and then cooled to 0-5 °C under ice-water bath, and then sodium hydride is added into the reaction vessel in batches for reaction, and after the reaction is carried out for a set time, raw material 6-1 are dropwise added into the reaction system for reaction; after the reaction is completed, quenching, extraction and liquid separation, drying, and purification are sequentially carried out, to obtain a colorless oily Compound 6.

15. The preparation method of the cationic lipid compound according to claim 23, wherein the chemical formula for preparing the Compound 23 is as follows: 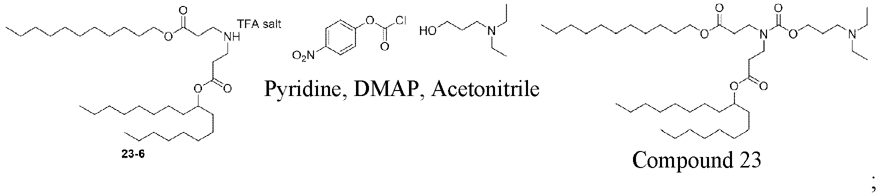 preferably, the preparation steps of the Compound 23 comprise: raw material 23-6, pyridine, 4-dimethylaminopyridine and acetonitrile are added into a reaction vessel under room temperature; after full dissolution, p-nitrophenyl chloroformate is added thereto for reaction, wherein the reaction is monitored by TLC, until a new fluorescent spot is generated; and then 3-dimethylamino-1-propanol is added into the reaction system for reaction overnight; after the reaction is completed, extraction and liquid separation are carried out, and the organic phase is washed, dried and purified, to obtain the Compound 23.

16. Use of the cationic lipid compound according to any one of claims 1-9 in the preparation of a delivery system for mRNA.

17. A delivery system for mRNA, wherein the delivery system for mRNA comprises the cationic lipid compound according to any one of claims 1-9.
